# EUROPEAN PATENT APPLICATION

(11) **EP 3 093 027 A1**
(43) Date of publication of application: **16.11.2016**
(21) Application number: 15167119.5
(22) Date of filing: 11.05.2015
(51) Int. Cl.: A61K 47/48, A61K 49/00, A61K 31/80, C08F 293/00, C08F 220/34, C08F 220/38

(54) **COPOLYMER COMPOSITIONS AND USES THEREOF**

(71) Applicant: ETH Zurich, 8092 Zürich ETH-Zentrum (CH); Universität Zürich, 8006 Zürich (CH); Augurix S.A., 1870 Monthey (CH)
(72) Inventor: Leroux, Jean-Christophe, 8053 Zürich (CH); Rogler, Gerhard, 8032 Zürich (CH); Luciani, Paola, 8050 Zürich (CH); Estella, Ander, 1820 Montreux (CH); Anghel Ramond, Silvia, 74500 Lugrin (FR); Besson Duvanel, Cécile, 1815 Clarens (CH)
(74) Representative: reuteler & cie SA

(57) **Abstract**

The present invention is directed to non-biodegradable copolymer diagnosis agent that has a low affinity for the mucosa in general and allows the transportation of specific molecules that recognizes specific target biomarkers useful in the diagnosis of a certain number of disorders such as pulmonary, gynecologic, eye and gastrointestinal diseases or disorders, and certain conditions of those diseases (such as fibrosis and inflammation), in particular inflammatory bowel disease, such as Crohn's disease or ulcerative colitis (UC) as well as coeliac disease and cancers such as colorectal cancers.

SEQUENCE LISTING

<110> ETH Zürich
UNIVERSITÄT ZÜRICH
Augurix SA

<120> COPOLYMER COMPOSITIONS AND USES THEREOF

<130> P1851EP00

<160> 1

<170> PatentIn version 3.3

<210> 1
<211> 12
<212> PRT
<213> murine

<400> 1

Ser Trp Thr Leu Tyr Thr Pro Ser Gly Gln Ser Lys
1               5                   10

## Description

### Field of the invention

The present invention relates to a non-biodegradable copolymer diagnosis agent that has a low affinity for the mucosa in general and allows the transportation of specific targeting molecules which recognize specific biomarkers, useful in the diagnosis of pulmonary, gynecological, eye and gastrointestinal diseases or disorders, and certain conditions of those diseases (such as fibrosis and inflammation), in particular inflammatory bowel disease, such as Crohn's disease or ulcerative colitis (UC) as well as coeliac disease and cancers such as colorectal cancers.

### Background of the Invention

Hydroxyethyl methacrylate (HEMA) is a widely used monomer as a component of dental bonding resins and toxicity studies in guinea pigs have demonstrated its low toxicity when ingested over the oral route. The use of HEMA has been very used for gastrointestimal (GI) administration. Lehr et al., 1990, Journal of Controlled Release 13, 51-62 performed a study in which microparticles made of polymerized HEMA were coated with different mucoadhesive polymers and further administered, while Pinier et al., 2012, Gastroenterology 142, 316-325 developed a copolymer of HEMA and 4-styrene sulfonic acid sodium salt hydrate (SS) (copolymer P(HEMA-co-SS which is reported as binding to gluten and reducing immune response in gluten-sensitized mice and human tissues) for its oral administration with no signs of toxicity.
HEMA and sulfopropyl methacrylate (SPMA) have been used for the last years in the development of electroconductive non-toxic hydrogels as blends of poly(HEMA-co-PEGMA-co-HMMA-co-SPMA) and poly(Py-co-PyBA) (Justin et al., 2009, Biomacromolecules 10, 2539-2549*).*

Further, pharmaceutical compositions comprising polymeric binders in the form of copolymers of HEMA and SS or copolymers of HEMA and SPMA potassium salt and with non-hydrolysable covalent bonds have been developed in particular for the treatment of celiac disease (WO 2007/053935). Those copolymers have been said to form electrostatic bonds at a pH lower than the isoelectric point of gluten and peptides derived from the degradation of gluten and are being able to bind to gluten or peptides derived from the degradation of gluten in the GI tract. ω-azido oligoethyleneglycol methacrylate (AOEGMA) is a monomer composed of a methacrylate moiety connected to a short poly(ethylene)glycol (PEG) chain, as described by Lutz, 2008, J. Pol. Sci A., 46: 3459-3470*,* and an azide group in the extreme opposite to the methacrylate moiety.

In the field of diagnosis of gastrointestinal disorders, gynecological cancers or infections, respiratory diseases such as lung fibrosis or lung infections and ocular disorders such as autoimmune macular degeneration, there is a need for diagnostic agents which are non-toxic and which are not hydrolysable or absorbable by the organism. In particular, when the diagnostic agent is to be in contact with a mucous membrane, it is of importance that this agent does not present a non-specific adhesion to the mucous membrane, so that the entire administered dose reaches the target area.

Disorders that affect the female reproductive system are known as gynecologic disorders. Pelvic pain, vaginal discharge, vaginal itching, abnormal vaginal bleeding, and breast pain and lumps are amongst the most common symptoms of gynecologic disorders. These symptoms may be due to infections by yeasts or bacteria that can cause inflammation of different parts of the reproductive system. However, they might also be related to age related hormonal changes; therefore, the significance of these symptoms often depends on the woman's age. Cervical, endometrial and ovarian cancers are the most common cancers of the female reproductive system. Among all of these reproductive-system cancers, early detection is crucial. But detection can be a very difficult task, especially in the early stages. Current treatments for all three cancers, especially in advanced stages, include surgery followed by chemotherapy or a combination of chemo and radiation therapies. The exact mix of the cancer-fighting drugs, sometimes called a "cocktail," depends on the particular form and stage of the cancer. That is why an early detection of these disorders is important, as the treatment will often be simpler and more likely to be effective.

Pulmonary infections can lead to pulmonary fibrosis, or lung fibrosis, which is a respiratory disease in which scars are formed in the lung tissues, leading to serious breathing problems. Scar formation, the accumulation of excess fibrous connective tissue, leads to thickening of the walls, and causes reduced oxygen supply in the blood. As a consequence, patients suffer from perpetual shortness of breath. There is no known cure for the scars and damage in the lung due to pulmonary fibrosis. Early diagnosis of these infections or fibrotic disorders would increase the quality of life of these patients.

Age-related macular degeneration (AMD) is a disorder that usually affects older adults and results in a loss of vision in the center of the visual field (the macula) because of damage to the retina. The retina is a network of visual receptors and nerves. It lies on the choroid, a network of blood vessels that supplies the retina with blood. Recent findings indicate that autoimmunity is intimately involved in the formation of yellow, extracellular deposits called drusen, which accumulate in the retina. It is a major cause of blindness and visual impairment in older adults (>50 years). Macular degeneration can make it difficult or impossible to read or recognize faces, although enough peripheral vision remains to allow other activities of daily life. Two forms of AMD are known: the dry and the wet form. In the dry (nonexudative) form of AMD, drusen accumulate between the retina and the choroid, and the retina can become detached. In the wet (exudative) form, which is more severe, blood vessels grow up from the choroid behind the retina, and the retina can also become detached. It can be treated with laser coagulation, and with medication that stops and sometimes reverses the growth of blood vessels. In the recent years, there has been a proactive position of ophthalmologists defending that an early detection and treatment of neovascular AMD results in a better visual outcome.

Disorders that affect the GI tract are called digestive disorders. Some disorders simultaneously affect several parts of the digestive system, whereas others affect only one part or organ. Abdominal pain, cramping, bloating, constipation, diarrhoea are all very common clinical symptoms that generate more than 70 million physician visits annually in Europe and over 94 million in the United States. Usually, patients suffering from these symptoms consult at the primary care, where physicians will need to discriminate patients with a benign functional GI disorder, typically irritable bowel syndrome (IBS) from those suffering from an organic disorder, either with inflammatory etiology (Crohn's disease (CD) or ulcerative colitis (UC), under the umbrella term inflammatory bowel disease, IBD), autoimmune etiology (celiac disease), infectious or oncology etiology.

CD and UC are collectively referred to as IBDs and usually develop in individuals having an impaired immunity, or exposed to environmental factors such as smoking, diet and oral contraception or in genetically susceptible individuals, resulting in a chronic inflammation of different parts of the GI tract due to the attack of the body's own immune system. The prevalence of IBDs has increased in most developed countries during the past century and the biological hallmarks of active IBD are pathological changes in homeostasis of the intestinal epithelium characterized by altered proliferation, increased inflammatory gene expression and apoptosis and reduced differentiation of intestinal epithelial cells.

UC affects the mucosa of the colon and rectum whereas CD affects any segment of the GI tract and presents patchy transmural inflammation where deeper layer of the mucosa are affected.

Patients with mild IBD receive 5-aminosalicylates, antibiotics or topical corticosteroids such as budesonide, and those with moderately active or severe disease, or acute flares, are treated with systemic corticosteroids. More aggressive therapeutic strategies involving the use of immunosuppressives, like thiopurines (azathioprine or 6-mercaptopurine) and/or biologics targeting tumor necrosis factor (TNF-α) are currently reserved for steroid-refractory patients and surgery is indicated for patients failing biologics. Increasing evidence indicates that early intervention with immunosuppressives and/or anti-TNF biologics such as infliximab, adalimumab, certolizumab pegol can confer rapid and prolonged benefits and lead to a high rate of remission in active and severe CD, reducing notably the need for hospitalization and surgery. Such early aggressive treatment also called "top-down" appears to be a disease-modifying treatment. However, recent studies seem to indicate that if anti-TNF therapies may change the natural course of IBD by decreasing the need for surgery in both CD and UC patients, serious side effects (increased lymphoma risk, reactivation of latent tuberculosis, adverse reactions during infusion, potential risk of immunogenicity and less efficacy if restarted) are common. Therefore, there is an important need for stratification of IBD cases and a need for diagnosis tools allowing an early diagnosis which will also allow a fine diagnosis and distinction between an intermittent non-evolutive inflammation and a chronic inflammation which will lead to a fibrotic evolution, in view of the early selection of an adequate type of treatment.

Today, good predictors of the severity of digestive disorders and their outcome are still lacking in current medical practice. The state-of-the-art is still based on endoscopic procedures and several publications have demonstrated increasing evidence that endoscopy parameters are better predictors to help identifying patients who should be treated with more aggressive therapies (Thomson, 2009, Red Book, Pharmacy's Fundamental Reference, 111th edn. (Thomson PDR, Montvale, 2009). The most recent advances are in the field of confocal laser endomicroscopy (CLE), a technique that allows real time *in vivo* histology of 1'000-fold magnification during on-going endoscopy. Endomicroscopy requires the application of fluorescent agents either systemically (fluorescein) or topically (acriflavin, cresyl violet). For instance, in case of IBD, this technique offers many advantages, the main one being that it provides information equivalent to histopathology (Allez et al., 2010, World J. Gastroenterol., 16, 2626-2632). It bridges the traditional gap between macroscopic *in vivo* tools such as magnetic resonance imaging and positron emission tomography that have limited spatial resolution, with high-resolution microscopy, formerly limited to *ex vivo* samples. However, the fluorescent agents currently used are non-specific, thereby limiting the ability to discriminate the different disorders. As a result, this discrimination still relies very much on the know-how and experience of the physician performing the procedure.

In particular, in the field of diagnosis of gastrointestinal disorders, in particular for oral diagnosis purpose, there is a further challenge in a need for diagnostic agent having a low affinity for the non-diseased parts of the GI tract, in particular mucins.

As a result, an important challenge in medicine is to find more effective ways of diagnosing and treating these disorders. One of the challenges to be overcome relates to the ability to administer imaging agents, therapeutic agents and ligands "site-specific targeted" such that they selectively reach the desired targets in each disorder. Therefore, there is an important need of finding a way to provide the physician performing the procedure with a mean to visually highlight and guide straight to the mucosal surface spots and discriminate the form and etiology of the gastrointestinal disorder, which would result in shorter endoscopic procedures, thereby minimizing the risks to the patient, as well as its overall cost.

### Summary of the invention

The invention relates to the unexpected findings of that the existence of azide groups in the monomer AOEGMA offers excellent opportunities for attaching different molecules that will be responsible for the recognition of a biological marker and advantageous properties when combined with other monomers for the development of a polymeric structure. In particular, the invention relates to a new copolymer of methacrylate based monomers, comprising at least one azide bearing methacrylate monomer together with at least one monomer selected from sulfonated methacrylate monomer and hydroxylated methacrylate monomer (e.g. a copolymer of hydroxyethyl methacrylate (HEMA), 3-sulfopropyl methacrylate (SPMA) and ω-azido oligoethyleneglycol methacrylate (AOEGMA)) which is non-biodegradable, has a low affinity for the mucosa in general and in particular for the GI tract and allows the attachment of a fluorescent dye and one or more targeting molecules which recognize specific biomarkers, depending on the disease to diagnose. In particular, the copolymer of the invention has the advantage of being not absorbable, resist digestion in the gut, bind with high specificity and affinity to the disorder site due to the specific target molecule that recognizes a biomarker, and can be easily manufactured. It also protects the employed target molecules from GI degradation when those are of peptidic nature and allows linking several copies of the target molecules for increasing the affinity of the polymer as diagnosis probe. The copolymer of the invention can be a tailored probe, depending on the disease to diagnose and will allow to render the endoscopic examination less labour intensive and more patient-friendly, while opening new horizons in terms of diagnosis and timely treatments.

A first aspect of the invention provides a block copolymer of methacrylate based monomers comprising at least one azide bearing methacrylate monomer (e.g. Formula M3) and at least one another monomer selected from sulfonated methacrylate monomer (e.g. Formula M1) and hydroxylated methacrylate monomer (e.g. Formula M2), such as for example a block copolymer of ω-azido oligoethyleneglycol methacrylate (AOEGMA) with at least another monomer selected from 2-sulfoethyl methacrylate (SEMA) and 3-sulfopropyl methacrylate (SPMA).

Another aspect of the invention relates to a pharmaceutical composition comprising at least one block copolymer of the invention and at least one pharmaceutically acceptable carrier, diluent or excipient thereof

Another aspect of the invention relates to a use of a block copolymer of the invention for the preparation of a medical composition for the diagnosis or prevention and/or treatment of disease or disorder, and in particular of inflammatory bowel diseases (IBDs), in particular Crohn's disease (CD) and ulcerative colitis (UC) as well as coeliac disease and cancers such as a colorectal cancer.

Another aspect of the invention relates to a method of detecting, preventing, repressing or treating a disorder or a disease, in particular inflammatory bowel diseases (IBDs), in particular Crohn's disease (CD) and ulcerative colitis (UC), as well as coeliac disease and cancers such as a colorectal cancer, in a subject, said method comprising administering in a subject or contacting a tissue in said subject in need thereof an effective amount of at least one block copolymer of the invention or a pharmaceutical formulation thereof.

Another aspect of the invention relates to a method of preparation of a diagnosis probe comprising the step of coupling a block copolymer of the invention with a targeting molecule that recognizes a specific biomarker.

Another aspect of the invention relates to a diagnosis probe according to the invention, formulations thereof and its use in the diagnosis and prevention of IBD.

Another aspect according to the invention provides a diagnostic composition comprising a block copolymer or a diagnosis probe according to the invention and at least one pharmaceutically acceptable carrier, diluent or excipient thereof.

Another aspect of the invention relates to a pharmaceutical formulation comprising at least one block copolymer of the invention combined with at least one co-agent useful in the treatment of inflammatory bowel diseases (IBDs), in particular Crohn's disease (CD) and ulcerative colitis (UC), as well as coeliac disease and cancers such as a colorectal cancer, and at least one pharmaceutically acceptable carrier, diluent or excipient thereof.

Another aspect of the invention relates to the use of a molecular targeting agent according to the invention for the diagnosis or treatment of a disease or a disorder. Another aspect of the invention relates to a kit for the detection of a disease or disorder comprising a molecular targeting agent of the invention or a diagnosis probe according to the invention.

### Description of the figures

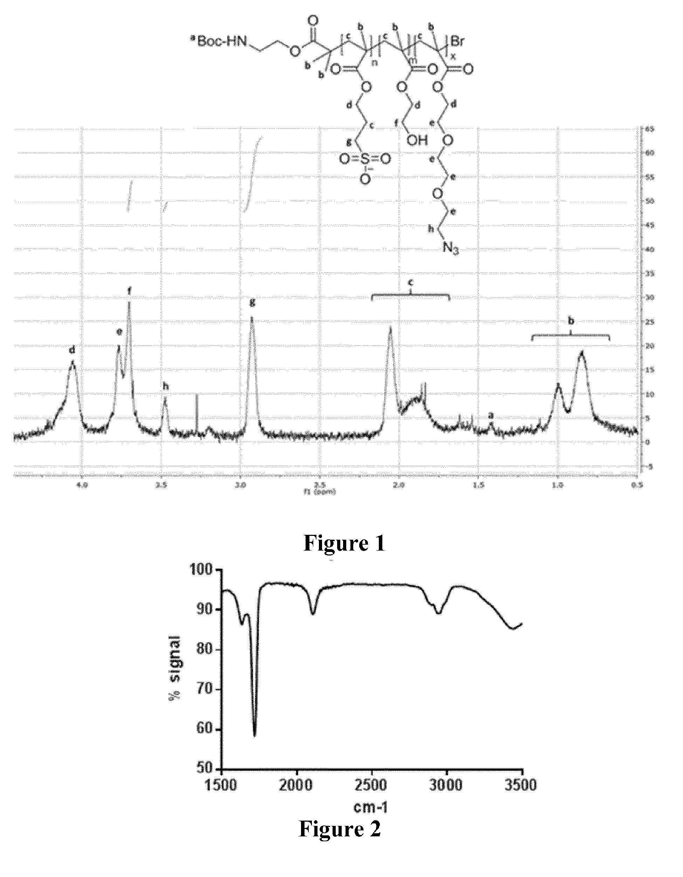

**Figure 1** shows the ¹H-NMR of a polymer of the invention wherein the SPMA:HEMA:AOEGMA is 68:23:9 as described in Example 6.
**Figure 2** shows the FTIR spectrum of a polymer of the invention wherein the SPMA:HEMA:AOEGMA is 68:23:9 as described in Example 6 and the typical azide peak at 2'100cm⁻¹.

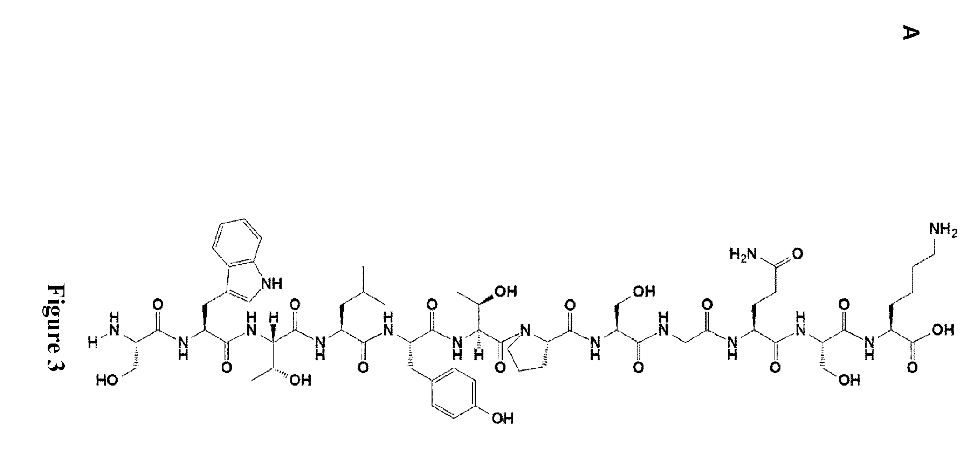

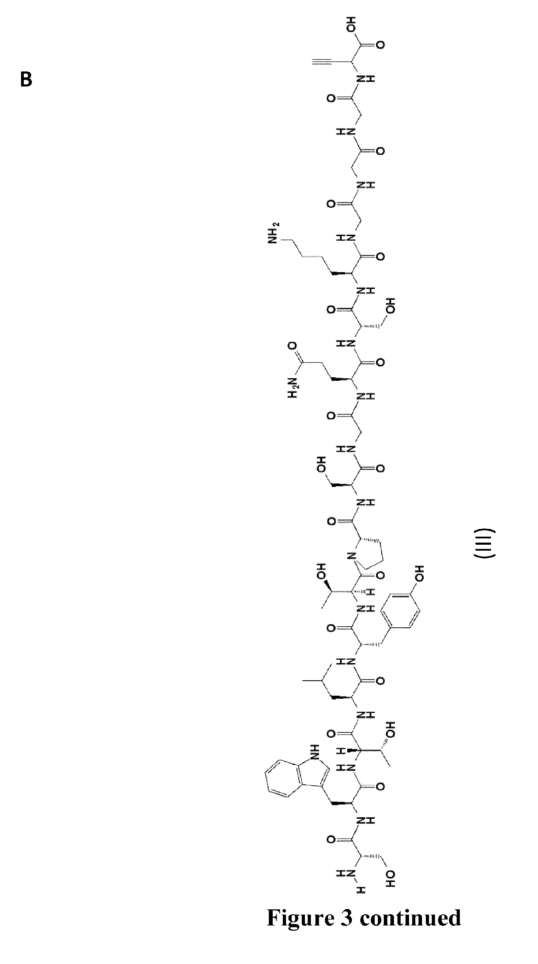

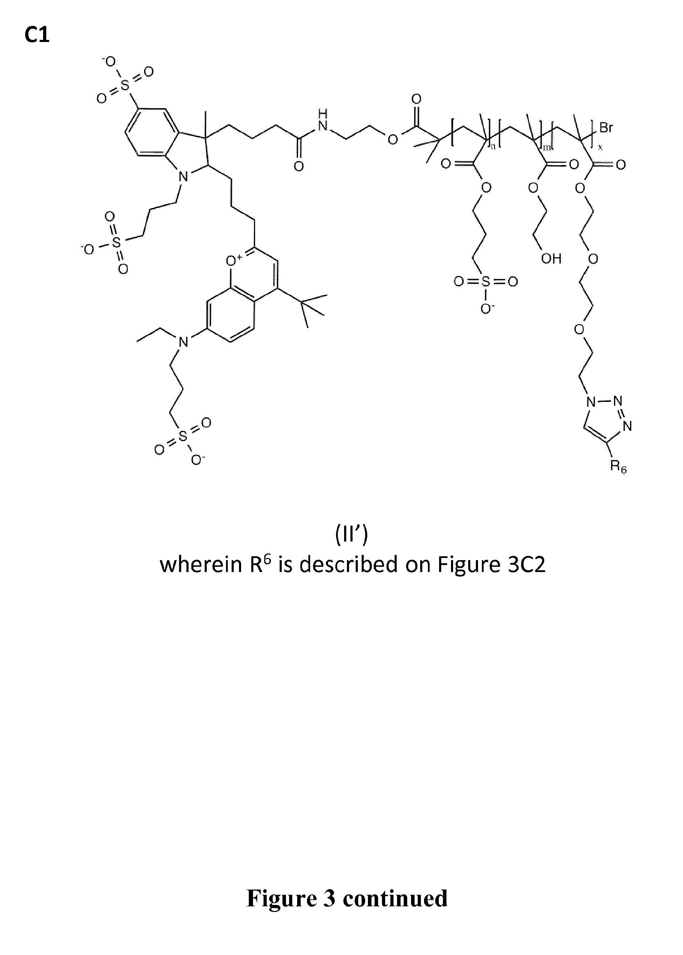

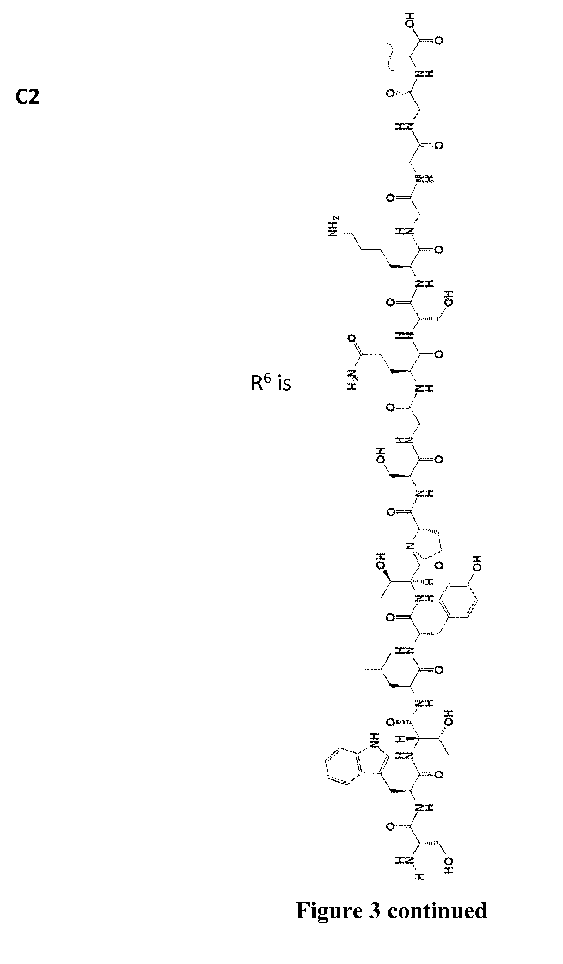

**Figure 3** shows the structures of molecular targeting peptide of SEQ ID NO: 1 used in Example 6 (Fig.3A), the structure of the corresponding alkyne-bearing targeting molecule (Fig. 3B) used for coupling to the labelled polymer of Formula (III) and of the diagnosis probe polymer of the invention comprising a NIR dye and a molecular targeting peptide of SEQ ID NO: 1 of Formula (II') (Fig. 3C1), wherein R⁶ is represented on Figure 3C2.

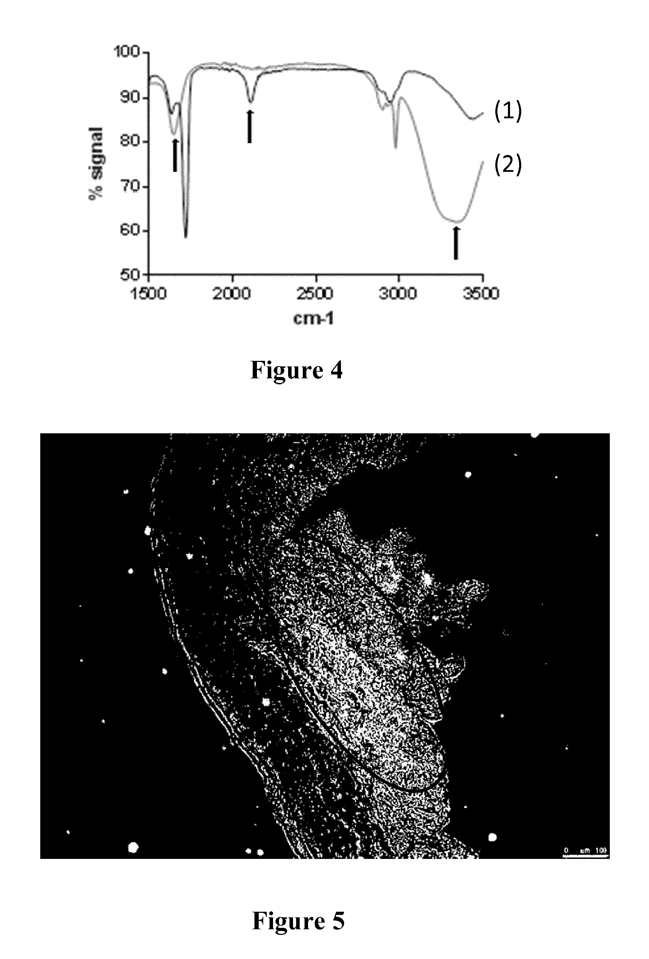

**Figure 4** shows the FTIR spectra of a labelled polymer of Example 6 before the coupling to a molecular targeting peptide with the click reaction (1) and after the coupling with the molecular targeting peptide (2) of Example 6. The azide peak at 2'100 cm⁻¹ disappears (middle arrow) and two broad peaks corresponding to amide bonds of the peptide at around 1'700 and 3'250 cm⁻¹ appear (left and right arrows).
**Figure 5** shows a NIR microscopy image of inflamed mouse colon tissue incubated with the probe at a concentration of 5 µM dye. White circle shows the selective binding to inflamed regions of the probe.

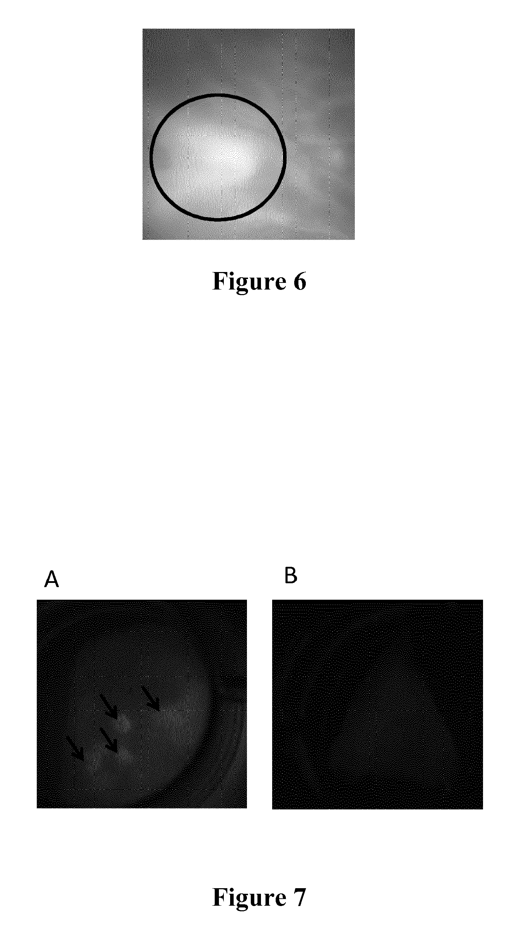

**Figure 6** shows the stereomicroscope image of human colon obtained from a colorectal cancer patient and further incubated with the probe at a 100 µM dye concentration as described in Example 9. Targeting molecule used in the probe is inflammation specific. Circle shows the selective binding of the probe to the target.
**Figure 7** shows the stereomicroscope images of swine colon sections incubated with a 100 µM dye concentration of A) a polymer of the invention bearing a targeting molecule for the extracellular domain of the cystic fibrosis transmembrane conductance regulator protein (CFTR); and B) a polymer of the invention without any targeting molecule, used as negative control as described in Example 8. Arrows in A show the selective binding of the probe to the target. B image shows the lack of unspecific binding of the untargeted polymer of the invention.

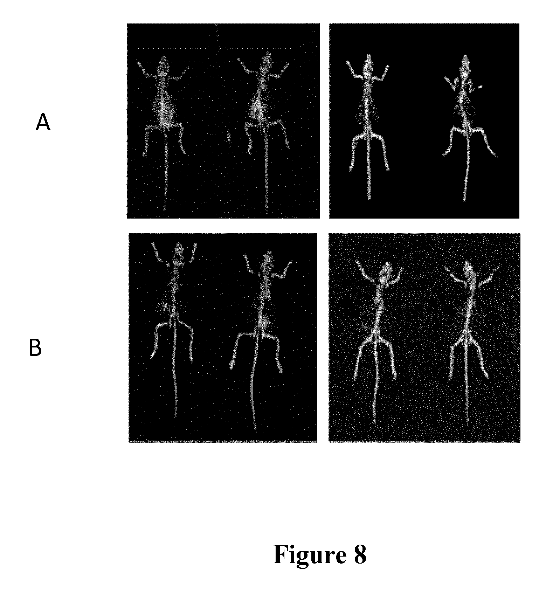

**Figure 8** shows the SPECT-CT images of both healthy (A) and inflamed mice (B) after 4 h (left) and 24 h (right) of the oral administration of ^{99m}Tc-labeled probe as described in Example 10 (white arrows show residual activity of the probe 24 h after its administration to inflamed mice).

### Detailed description

The term "Inflammatory Bowel Disease" (IBD) refers to a group of inflammatory conditions of the colon and small intestine. The main forms of IBD are Crohn's disease (CD) and ulcerative colitis (UC). The main difference between CD and UC is the location and nature of the inflammatory changes. CD can affect any part of the gastrointestinal tract, from mouth to anus, although a majority of the cases start in the terminal ileum. UC, in contrast, is restricted to the colon and the rectum. Both disorders may present with any of the following symptoms: abdominal pain, vomiting, diarrhoea, rectal bleeding, severe internal cramps/muscle spasms in the region of the pelvis, and weight loss. Anemia is the most prevalent extra-intestinal complication of inflammatory bowel disease. Patients of IBD have an increased risk of colorectal cancer but since the disorder is usually caught much earlier than the general population in routine surveillance of the colon by colonoscopy, patients are much more likely to survive. New evidence suggests that patients with IBD may have an elevated risk of endothelial dysfunction and coronary artery disease. IBD diagnosis is generally made by assessment of inflammatory markers (such as fecal calprotectin, fecal lactoferrin or calgranulin C) in stool in conjunction with imaging technologies such as optical, ultrasound, magnetic resonance imaging (MRI), X-ray, computer tomography (CT), position emission tomography (PET), single photon emission computed tomography (SPECT) and colonoscopy with biopsy of pathological lesions. The group of IBDs further includes lymphocytic colitis, ischemic colitis, Bechet's disease, diversion colitis, and irritable bowel syndrome.

"Coeliac disease" is an autoimmune disorder of the small intestine that occurs in genetically predisposed people of all ages from middle infancy onwards. Symptoms include pain and discomfort in the digestive tract, chronic constipation and diarrhea, failure to thrive (in children), anaemia and fatigue, but these may be absent, and symptoms in other organ systems have been described. Vitamin deficiencies are often noted in people with coeliac disease owing to the reduced ability of the small intestine to properly absorb nutrients from food. Coeliac disease is caused by an immunological reaction to gliadin, a prolamin (gluten protein) found in wheat, and similar proteins found in the crops of the tribe Triticeae (which includes other common grains such as barley and rye). Upon exposure to gliadin, and specifically to three peptides found in prolamins, the enzyme tissue transglutaminase modifies the protein, and the immune system cross-reacts with the small-bowel tissue, causing an inflammatory reaction. This interferes with the absorption of nutrients because the intestinal villi are responsible for absorption. The only known effective treatment is a lifelong gluten-free diet.

The term "biocompatible dye" refers to a biocompatible agent, namely an agent which does not induce severe side effects or reactions in the host, which allows imaging and/or treating tissues it is bound to. This term includes NIR, non-NIR dye that emit above the orange-red and photodynamic therapy (PDT) dyes. Near-infrared (NIR) fluorescence imaging is a dynamic diagnostic method for detecting pathologic processes at the cellular and molecular levels in vivo, with the help of development of target-specific probes. The use of NIR fluorescent light (650-2500 nm) has several advantages for imaging organisms and tissues which exhibit low absorption and autofluorescence in the NIR spectral range. Therefore, the use of NIR fluorescent dyes grafted on the polymers of the invention affords high tissue penetration due to reduced endogenous absorbers (water, oxygenated hemoglobin, and melanin) and relatively low autofluorescence (Frangioni, 2008, J. Clin. Oncol., 26, 4012-4021) which minimizes background interference. Further, NIR fluorescence imaging potentially has high sensitivity and restricted technical requirements and is relatively inexpensive, and presents a low risk to living organisms due to the use of nonionizing radiation. Therefore, according to one particular aspect, a biocompatible dye according to the invention is a NIR fluorescent dye such as described in He et al., 2010, Trends Mol. Med. 16, 574-583 and Wunder et al., 2009, Neuroscience 158, 1161-1173*.* According to a particular aspect, NIR fluorescent dyes according to the invention include cyanine dyes and their derivatives (DyLight™, IRDye™), squaraine, phthalocyanines, porphyrin derivatives and BODIPY (borondipyrromethane) analogues such as described in Luo et al., 2011, Biomaterials 32(29); 7127 - 7138. According to a particular embodiment, NIR fluorescent cyanine dyes include DyLight™ 680 or maleimide or *N*-Hydroxysuccinimide derivatives thereof and indocyanine green (ICG) [sodium 4-[2-[(1*E*,3*E*,5*E*,7*Z*)-7-[1,1-dimethyl-3-(4-sulfonatobutyl)benzo[*e*]indol-2-ylidene]hepta-1,3,5-trienyl]-1,1-dimethylbenzo[*e*]indol-3-ium-3-yl]butane-1-sulfonate] :

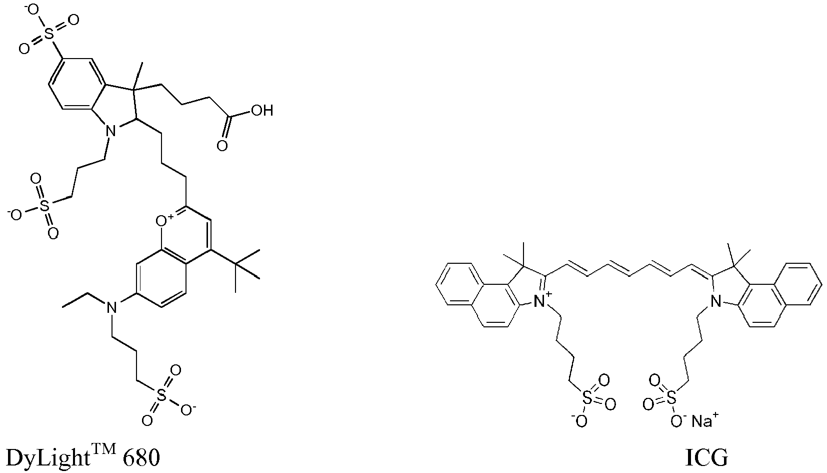

ICG is used in clinical studies in human for diagnostics investigations and it can be used both for diagnostics and therapy (theranostics).

According to another aspect, biocompatible dyes according to the invention include non-NIR dyes that emit at the orange-red or above (>600 nm), for example like the DyLight 633, Europium (III) complexes or peridinin, a light-harvesting carotenoid, which presents a wide absorption range starting at around 440 nm and shows an emission maximum at around 680 nm, when complexed with chlorophyll and a binding protein.

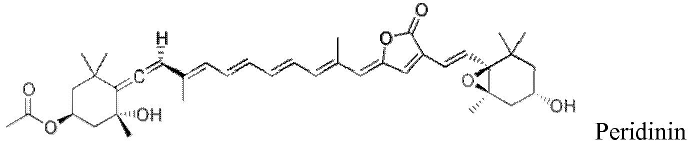

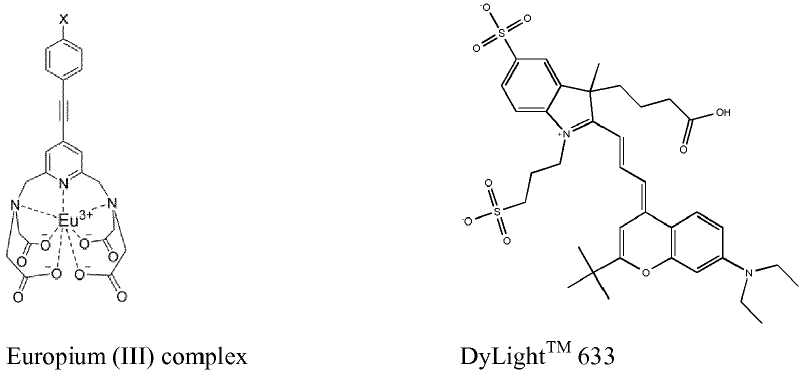

According to another aspect, biocompatible dyes according to the invention include rhodamine derivatives that can emit at around 552 nm, in particular 5/6-Carboxyrhodamine 6G, since they present high quantum yield and extinction coefficient and could be used with the currently available lasers in clinic.

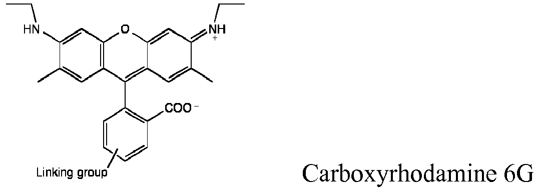

According to another aspect, biocompatible dyes according to the invention include rhodamine derivatives that can emit at 580 nm, in particular the carboxytetramethylrhodamine (TAMRA).

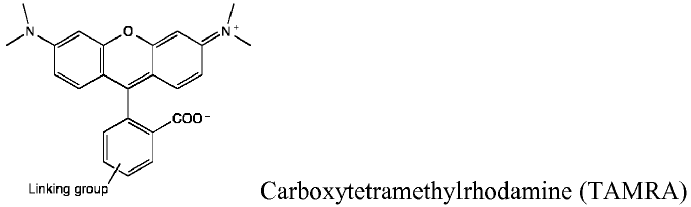

According to another aspect, biocompatible dyes according to the invention include rhodamine derivatives that can emit at 615 nm, in particular the sulforhodamine 101 (Texas Red^{®}).

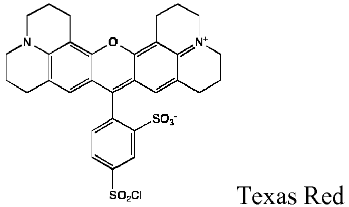

Photodynamic therapy (PDT) dyes are dye sensitizers for photodynamic therapy and include dyes such as described in Ormond et al., 2013, Materials, 6, 817-840*,* in particular ICG, IRDye™ 800 and BODIPY derivatives. PDT dyes are useful as a theranostic agent or in theranostic therapy.

As used herein, the term "targeting molecule" is a molecular or biological entity that selectively recognizes a biomarker in the body, which can be measured or otherwise evaluated as an indicator of a normal biologic process, disease state, or response to a therapeutic intervention. The biomarkers that are aimed to be targeted with the targeting agent of the invention include an RNA transcript, a polypeptide, a protein, or a modified protein such as a glycoprotein, the presence or absence of which are predictive of a disorder or of the status of progression of a disorder or disease. Targeting molecules for biomarkers according to the invention which are disorder- or disease-specific can be used to detect or monitor the progression of a disorder (e.g. cancer or pre-cancerous conditions, from mild to chronic/fibrotic colon inflammation), as well as to distinguish those conditions from each other and from benign (e.g. non-cancerous, intermittent, non-evolutive inflammation) disease states. The targeting molecules can be used as individual molecules as well as a panel of different molecules useful for detect or monitor the progression of a disorder. Examples of biomarkers aimed to be targeted by targeting molecules and diagnosis probes of the invention include, for example, biomarkers of IBD (from mild to chronic inflammation, including the development of further neoplasia) such as those described in Letellier et al., 2014, British Journal of Cancer, 111, 726-735*.*

As used herein, the term "polypeptide" is used in its conventional meaning, i.e., as a sequence of amino acids. The polypeptides are not limited to a specific length of the product; thus, peptides, oligopeptides, and proteins are included within the definition of polypeptide, and such terms may be used interchangeably herein unless specifically indicated otherwise. This term also does not refer to or exclude post-expression modifications of the polypeptide, for example, glycosylations, acetylations, phosphorylations and the like, as well as other modifications known in the art, both naturally occurring and non-naturally occurring. A polypeptide may be an entire protein, or a subsequence thereof.

The term "fragments" refers to polypeptides comprising a portion of peptide sequence corresponding to contiguous amino acids of a polypeptide set forth herein, including all intermediate lengths and variants thereof.

The term "variant" can apply to either a polynucleotide or a polypeptide. A polypeptide "variant," as the term is used herein, is a peptide or a polypeptide substantially homologous to the referenced peptide sequence, but which has an amino acid sequence different from that of the referenced. Such variants may be naturally occurring or may be synthetically generated, for example, by modifying one or more of the above polypeptide sequences of the invention described herein using any of a number of techniques well known in the art. In many instances, a variant will contain conservative substitutions. Substantially homologous means a variant amino acid sequence which is identical to the referenced peptide sequence except for the deletion, insertion and/or substitution of a few amino acids, e.g. 1, 2, 3, 4, 5, or 6 amino acids. Substantially homologous means a variant amino acid sequence that is at least 80%, at least 85%, at least 90%, at least 95%, at least 96%, at least 97%, at least 98% or at least 99% identical to the referenced amino acid sequence. A variant nucleic acid sequence can be at least 80%, at least 85%, at least 90%, at least 95%, at least 96%, at least 97%, at least 98% or at least 99% identical to the referenced nucleic acid sequence. The identity of two amino acid sequences or of two nucleic acid sequences can be determined by visual inspection and/or mathematical calculation, or more easily by comparing sequence information using known computer program used for sequence comparison such as Clustal package version 1.83.

A variant may comprise a sequence having at least one conservatively substituted amino acid. A "conservative substitution" is one in which an amino acid is substituted for another amino acid that has similar properties, such that one skilled in the art of peptide chemistry would expect the secondary structure and hydropathic nature of the polypeptide to be substantially unchanged (e.g. having similar physicochemical characteristics). Modifications may be made in the structure of the polynucleotides and polypeptides of the present invention and still obtain a functional molecule that encodes a variant or derivative polypeptide with desirable characteristics according to the invention. When it is desired to alter the amino acid sequence of a polypeptide to create an equivalent, or even an improved, variant or portion of a polypeptide of the invention, one skilled in the art will typically change one or more of the codons of the encoding DNA sequence. In making such changes, the hydropathic index, polarity, charge, solubility, hydrophobicity, hydrophilicity and/or the amphipathic nature of the amino acids are considered. The importance of the hydropathic amino acid index in conferring interactive biologic function on a protein is generally understood in the art (Kyte, et al., 1982, J. MoI. Biol., 157: 105-131). Examples of conservative substitutions include substitution of one aliphatic residue for another, such as Ile, Val, Leu, or Ala for one another, or substitutions of one polar residue for another, such as between Lys and Arg; Glu and Asp; or Gln and Asn. Other such conservative substitutions, for example, substitutions of entire regions having similar hydrophobicity characteristics, are well known (*Kyte, et al, 1982, supra*). For example, a "conservative amino acid substitution" may involve a substitution of a native amino acid residue with a non-native residue such that there is little or no effect on the polarity or charge of the amino acid residue at that position. Desired amino acid substitutions (whether conservative or non-conservative) can be determined by those skilled in the art at the time such substitutions are desired. Exemplary amino acid substitutions are presented in Table 1 below. The term "variant" also includes a peptide or polypeptide substantially homologous to the referenced peptide sequence, but which has an amino acid sequence different from that of the referenced sequence because one or more amino acids have been chemically modified or substituted by amino acids analogs. This term also includes glycosylated polypeptides.

**Table 1**

| **Original residues** | **Examples of substitutions** |
|---|---|
| Ala (A) | Val, Leu, Ile |
| Arg (R) | Lys, Gln, Asn |
| Asn (N) | Gln |
| Asp (D) | Glu |
| Cys (C) | Ser, Ala |
| Gln (Q) | Asn |
| Glu (E) | Asp |
| Gly (G) | Pro, Ala |
| His (H) | Asn, Gln, Lys, Arg |
| Ile (I) | Leu, Val, Met, Ala, Phe |
| Leu (L) | Ile, Val, Met, Ala, Phe |
| Lys (K) | Arg, Gln, Asn |
| Met (M) | Leu, Ile, Phe |
| Phe (F) | Leu, Val, Ile, Ala, Tyr |
| Pro (P) | Ala, Gly |
| Ser (S) | Thr, Ala, Cys |
| Trp (W) | Phe, Tyr |
| Thr (T) | Ser |
| Tyr (Y) | Trp, Phe, Thr, Ser |
| Val (V) | He, Met, Leu, Phe, Ala |

Generally, substitutions for one or more amino acids present in the original polypeptide should be made conservatively. Polypeptidic targeting molecules of the invention, fragments and variants thereof are capable of binding specifically to the target site of the disorder when administered *in vivo.*

Polypeptides of the invention are prepared using any of a variety of well-known synthetic and/or recombinant techniques, the latter of which are further described below. Polypeptides, portions and other variants generally less than about 150 amino acids can be generated by synthetic means, using techniques well known to those of ordinary skill in the art. In one illustrative example, such polypeptides are synthesized using any of the commercially available solid-phase techniques, such as the Merrifield solid-phase synthesis method, where amino acids are sequentially added to a growing amino acid chain (Merrifield, 1963, J. Am. Chem. Soc., 85:2149-2146).

The term "pharmaceutically acceptable" refers to a carrier comprised of a material that is not biologically or otherwise undesirable. It includes carriers suitable for therapeutic or diagnostic purposes.

The term "carrier" refers to any components present in a pharmaceutical formulation other than the active agent and thus includes diluents, binders, lubricants, disintegrants, fillers, coloring agents, wetting or emulsifying agents, pH buffering agents, preservatives, cryoprotectants and the like.

As used herein, "diagnosing" and the like generally mean the early detection of any change in one or more physiological parameters that are predictive of the occurrence of a disorder or of the precursor signs of a disease or disorder or of the status of progression of a disorder or disease.

As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a disease in a mammal, particularly a human, and is not necessarily meant to imply cure or complete abolition of symptoms, but refers to any type of treatment that imparts a benefit to a patient and includes: (a) preventing the disease from occurring in a subject which may be predisposed to the disease but has not yet been diagnosed as having it for example based on familial history, overweight status or age; (b) inhibiting the disease, i.e., arresting its development; or relieving the disease, i.e., causing regression of the disease and/or its symptoms or conditions such as improvement or remediation of damage.

In particular, prevention and/or treatment of IBD and coeliac disease according to the invention comprise to normalize or decrease the inflammatory conditions of the colon and small intestine of an individual. The term "treatment" refers to any type of treatment or prevention that imparts a benefit to a subject afflicted with or at risk of developing an inflammatory conditions of the colon and small intestine, including improvement in the condition of the subject (e.g., in one or more symptoms), delay in the onset of symptoms or slowing the progression of symptoms, etc.. According to one aspect, effects of a treatment according to the invention may be observed through one or more the following: generally improved well-being and improvement of symptoms, specifically of abdominal pain, vomiting, diarrhea, rectal bleeding, severe internal cramps/muscle spasms in the region of the pelvis, and weight loss and anemia. Treatment success may manifest itself in improved quality of life and reduced indications for surgery, as well as a reduced need for aggressive therapeutic strategies involving immunosuppressive thiopurines (azathioprine or 6-mercaptopurine) and/or biologics targeting tumor necrosis factor (TNF-α) or systemic steroids. Mucosal healing may be another sign of treatment success, along with a reduction in acute flares.

The term "subject" as used herein refers to mammals. For example, mammals contemplated by the present invention include human, primates, domesticated animals such as cattle, sheep, pigs, horses, laboratory rodents and the like.

The term "high-risk" IBD subjects or individuals are subjects that are at risk to develop inflammatory conditions of the colon and small intestine, in particular of developing Crohn's disease (CD) or ulcerative colitis (UC). Those include genetic predisposition such as a history of inflammatory conditions such as genetic (such as NOD2 gene variants), and personal or family history of IBDs. The risk or predisposition of developing inflammatory conditions of the colon and small intestine, in particular of developing Crohn's disease (CD) or ulcerative colitis (UC) can be evaluated by assessing personal and family history, and can be confirmed by calprotectin, lactoferrin or calgranulin C in stool in conjunction with imaging technologies such as optical, ultrasound, magnetic resonance imaging (MRI), X-ray, computer tomography (CT), position emission tomography (PET), single photon emission computed tomography (SPECT) and colonoscopy with biopsy of pathological lesions. The risk or predisposition of developing coeliac disease can be evaluated by assessing personal and family history, and can be confirmed by serological assessment of antibodies to the enzyme tissue transglutaminase (tTG).

The term "efficacy" of a treatment or method according to the invention can be measured based on changes in the course of disease or condition in response to a use or a method according to the invention. For example, the efficacy of a treatment or method according to the invention can be measured by measuring the level of stool calprotectin or calgranulin C in the subject before and after the treatment, as well as by colonoscopy and other imaging techniques. The most important indicator of efficacy is self-reported improvement of quality of life, and a reduction in the various symptoms listed above. The polymers of the invention and formulations thereof can be useful at diagnosing the onset or progression of IBD in high-risk individuals and in differentiating cases with intermittent, non-evolutive colon inflammation from those with or which will evolve into a chronic and fibrotic inflammation.

### Polymers of the invention

In one aspect, the present invention provides a block copolymer of methacrylate based monomers comprising at least one azide bearing methacrylate monomer (e.g. a monomer of Formula M3 as defined below) and at least one another monomer selected from sulfonated methacrylate monomer (e.g. a monomer of Formula M1 as defined below) and hydroxylated methacrylate monomer (e.g. a monomer of Formula M2 as defined below). 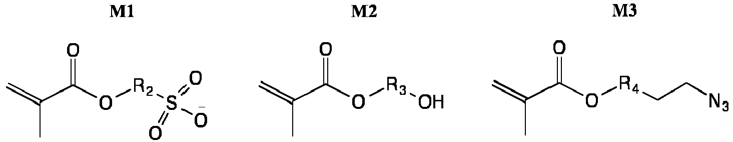 wherein R², R³ and R⁴ are independently selected from (CH₂)ₐ, benzyl and (CH₂CH₂O)_{b}, wherein a is an integer from 1 and 6, b is an integer from 1 and 8.

In one further aspect, the present invention provides a block copolymer of methacrylate based monomers comprising at least one azide bearing methacrylate monomer (e.g. a monomer of Formula M3) and at least one sulfonated methacrylate monomer.
In one another further aspect, the present invention provides a block copolymer of ω-azido oligoethyleneglycol methacrylate (AOEGMA) with at least another monomer selected from 2-sulfoethyl methacrylate (SEMA) and 3-sulfopropyl methacrylate (SPMA).

In one another further aspect, the present invention provides a block copolymer of ω-azido oligoethyleneglycol methacrylate (AOEGMA) with at least one SPMA monomer. In another further aspect, the present invention provides a block copolymer of ω-azido oligoethyleneglycol methacrylate (AOEGMA) with at least another monomer selected from 2-sulfoethyl methacrylate (SEMA) and 3-sulfopropyl methacrylate (SPMA) further comprising a monomer selected from hydroxyethyl methacrylate (HEMA) and hydroxypropyl methacrylate (HPMA).

In one further aspect, the present invention provides a copolymer of the invention of the following Formula (I): 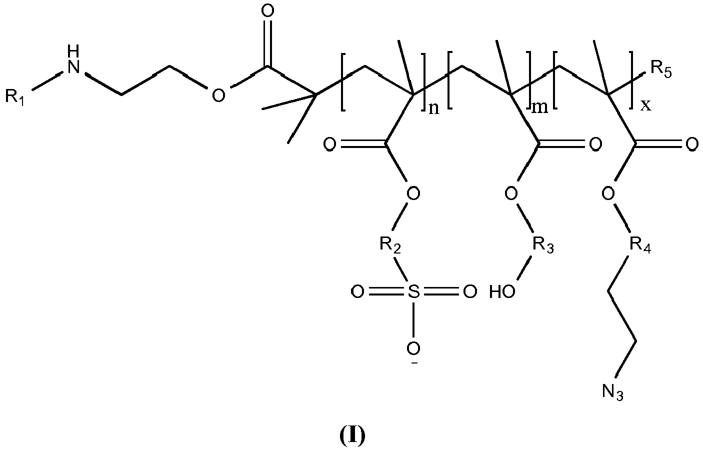 wherein n is an integer from about 20 and 550, m is an integer from about 0 and 1037 and x is an integer from 10 and 310, R¹ is selected from a blocking group such as t-butyloxy carbonyl (BOC), 9-fluorenylmethyloxycarbonyl (FMOC) or carboxybenzyl (Cbz) groups; N-hydroxysuccinimide (NHS) ester, a maleimide group, a hydrazide group, an amine group, a hydrogen atom and a dye; R², R³ and R⁴ are independently selected from (CH₂)ₐ, benzyl and (CH₂CH₂O)_{b}, wherein a is an integer from 1 and 6, for example 1 to 4 such as 2 or 3; b is an integer from 1 and 8, for example 1 to 6 such as 1, 2 or 3; and R⁵ is selected from an halogen (e.g. Br) and H.

In one aspect, the present invention provides a di- or tri-block copolymer of ω-azido oligoethyleneglycol methacrylate (AOEGMA) with at least another monomer selected from 2-sulfoethyl methacrylate (SEMA) and 3-sulfopropyl methacrylate (SPMA).

In a further aspect, the present invention provides a tri-block copolymer of ω-azido oligoethyleneglycol methacrylate (AOEGMA) with a monomer selected from 2-sulfoethyl methacrylate (SEMA) and 3-sulfopropyl methacrylate (SPMA) and with a monomer selected from hydroxyethyl methacrylate (HEMA) and hydroxypropyl methacrylate (HPMA).

In further particular aspect, the present invention provides a di- or tri-block copolymer of ω-azido oligoethyleneglycol methacrylate (AOEGMA) with 3-sulfopropyl methacrylate (SPMA) and optionally hydroxyethyl methacrylate (HEMA) and having an average molecular weight (Mn) from about 50 to about 500 kDa.

In a further aspect, is provided a copolymer of the invention of the following Formula (I) wherein R¹ is a biocompatible dye.

In a further embodiment, is provided a copolymer of the invention of the following Formula (I) wherein R¹ is a PDT dye.

In another further aspect, is provided a copolymer of the invention of the following Formula (I) wherein R¹ is a biocompatible dye selected from a NIR dye, a non-NIR dye emitting at the orange-red or above and a rhodamine derivative.

According to another further aspect, is provided a copolymer of the invention of the following Formula (I) wherein R¹ is a biocompatible NIR dye including cyanine dyes and their derivatives (such as DyLight™, IRDye™), squaraine, phthalocyanines, porphyrin derivatives and BODIPY (borondipyrromethane) analogues and indocyanine green.

According to another further aspect, is provided a copolymer of the invention of the following Formula (I) wherein R¹ is a biocompatible NIR dye selected from DyLight™ 680, DyLight™ 755, DyLight™ 800, IRDye™ 680, IRDye™ 700, IRDye™ 750, IRDye™800, Cy5.5™, Cy7™ and Cy7.5™.

According to another further aspect, is provided a copolymer of the invention of the following Formula (I) wherein R¹ is DyLight™ 680.

According to another further aspect, is provided a copolymer of the invention of the following Formula (I) wherein R¹ is a biocompatible non-NIR dye emitting at the orange-red or above.

According to another further aspect, is provided a copolymer of the invention of the following Formula (I) wherein R¹ is a biocompatible non-NIR dye emitting at the orange-red or above which is peridinin.

According to another further aspect, is provided a copolymer of the invention of the following Formula (I) wherein R¹ is the rhodamine derivative 5/6-carboxyrhodamine 6G.

According to another further aspect, is provided a copolymer of the invention of the following Formula (I) wherein R¹ is BOC.

According to another further aspect, is provided a copolymer of the invention of the following Formula (I) wherein R¹ is H.

According to another further aspect, is provided a copolymer of the invention of the following Formula (I) wherein R² is (CH₂)₃.

According to another further aspect, is provided a copolymer of the invention of the following Formula (I) wherein R³ is (CH₂)₂.

According to another further aspect, is provided a copolymer of the invention of the following Formula (I) wherein R⁴ is (CH₂CH₂O)₂.

According to another further aspect, is provided a copolymer of the invention of the following Formula (I) wherein R⁵ is Br.

According to another further aspect, is provided a copolymer of the invention of the following Formula (I) having Formula (Ic):

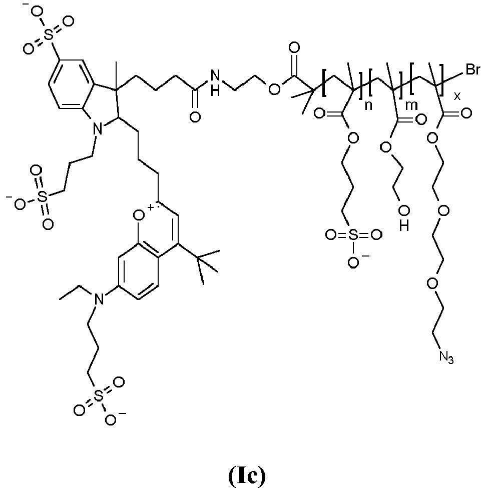

According to another further aspect, is provided a copolymer of the invention of the following Formula (I) having Formula (Ia).

According to another further aspect, is provided a copolymer of the invention of the following Formula (I) having Formula (Ib).

According to another further aspect, is provided a tri-block copolymer of the invention of the following Formula (I), wherein n is an integer from 50 to 300 (e.g. 246). According to another further aspect, is provided a tri-block copolymer of the invention of the following Formula (I), wherein m is an integer from 50 to 500 (e.g. 157). According to another further aspect, is provided a tri-block copolymer of the invention of the following Formula (I), wherein x is an integer from 10 to 50 (e.g. 33). According to another further aspect, is provided a tri-block copolymer of the invention of the following Formula (I), wherein the polymer has a molecular weight of about 50 kDa to about 150 kDa, typically from about 70 to about 140 such as for example from about 80 to about 130 kDa.

In a further aspect, is provided a copolymer of the invention of the following Formula (I) wherein the SPMA content is from about 5 to about 97% (weight/weight). In another further aspect, is provided a copolymer of the invention of the following Formula (I) wherein the HEMA content is from about 0 to about 90% (weight/weight). In another further aspect, is provided a copolymer of the invention of the following Formula (I) wherein the AOEGMA content is from about 3 to about 50% (weight/weight).

In another further aspect, is provided a tri-block copolymer of the invention of the following Formula (I), selected from polymers having the following weight ratios SPMA:HEMA:AOEGMA:

6:90:4 (MW: 51.9 kDa); 14:81:5 MW: 55.6 kDa); 39:58:3 (MW: 50.8 kDa); 63:34:3 (MW: 53.2 kDa); 68:23:9 (MW: 89 kDa); 77:20:3 (MW: 59.1 kDa); 91:6:3 (MW: 63.8 kDa) and 97:0:3 (MW: 58.6 kDa).

In another further aspect, is provided a tri-block copolymer of the invention of Formula (I) wherein n is 246, m is 157 and x is 33, wherein the polymer has a molecular weight of about 89 kDa.

In another further aspect, is provided a tri-block copolymer of the invention of Formula (I), having the following Formula (I') below:

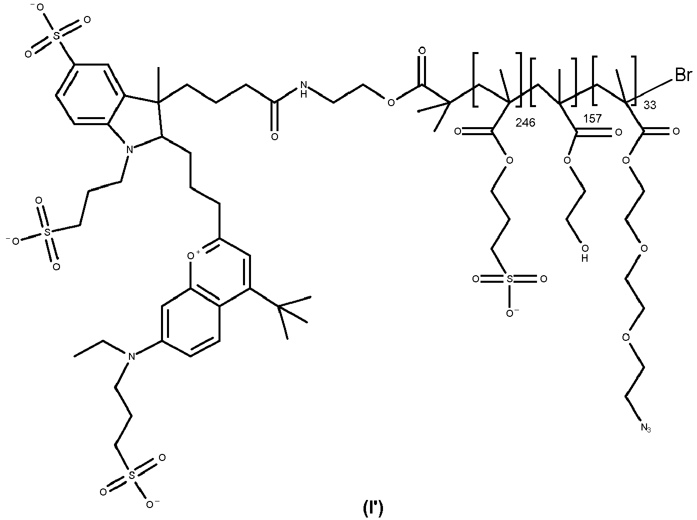

According to one aspect, the polymers of the invention present the advantage of not being absorbable, to resist digestion in the gut, bind with high specificity and affinity to the disorder site, and can be easily manufactured.

Further, the polymers of the invention can then be easily labeled with a bicompatible dye and the dye can then by delivered to the affected site through the polymer of the invention. The polymer of the invention is a methacrylate based polymer and methacrylate based polymers have been widely used in the past as food additives, therefore their safety when orally administered has been already proven and recognised by the European Food Safety Authority (FSA, Scientific Opinion on the safety of basic methacrylate copolymer for the proposed uses as a food additive, European Food Safety Authority Journal 2010, 8, 1513).

Even though poly(methacrylic acid) blocks are commonly used as precursors of anionic blocks, copolymers of the invention show little or no dependence on pH. Further, the polymers of the invention offer the advantage that the methacrylate monomers can be readily polymerized with controlled radical polymerization techniques such as ATRP polymerization and the presence of the azide group of the ω-azido oligoethyleneglycol methacrylate (AOEGMA) monomers allows the coupling of agents to interest, such as targeting molecules to the monomer by typical "click" reactions.

### Preparation of polymers of the invention

Polymers of the invention can be prepared by atom transfer radical polymerization (ATRP) of the monomers as described in Siegwart et al., 2012, Prog. Polym. Sci., 37, 18-37*,* in presence of an initiator molecule such as an alkyl halide as described in Matyjaszewski et al., 2012, Macromolecules, 45, 4015-4039*.* ATRP provides a simple route to many well-defined (co)polymers with predetermined molecular weight, narrow molecular weight distribution, and high degree of chain end functionality.

According to a particular aspect, a block copolymer of methacrylate based monomers comprising at least one azide bearing methacrylate monomer and at least one another monomer selected from sulfonated methacrylate monomer or hydroxylated methacrylate monomer according to the invention can be obtained by reacting, in presence of an alkyl halide initiator of Formula (ii), methacrylate based monomers of the invention, i.e. at least one sulfonated methacrylate monomer of Formula (M1), optionally in combination with at least one hydroxylated methacrylate monomer of Formula (M2) with an azide bearing methacrylate monomer Formula (M3), such as depicted under Scheme 1 below.

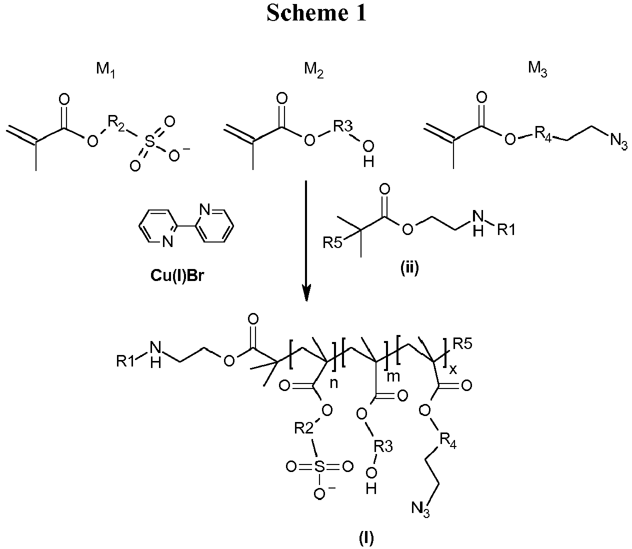

Sulfonated or hydroxylated methacrylate monomers according to the invention are commercially available or can be synthesized as described in US 2964557 and US 2877264. In particular, SPMA and HEMA monomers are commercially available or can be synthesized as described in Radugina et al., 1987, Bulletin of the Academy of Sciences of the USSR, Division of Chemical Science (English Translation), 36, # 10 p. 2083 - 2086*;* Park et al., 2004, Macromolecular Research, 12(4), 342-351*.*

Azide bearing methacrylate monomers according to the invention are commercially available or can be synthesized as described in Li et al., 2007, J Polym Sci A Polym Chem, 45 (18), 4300-4308*.*

In particular, AOEGMA monomer can be synthesized as provided in the present examples.

According to one aspect, the polymers of the invention are then obtained in the blocked form (R¹ being for example BOC, FMOC and Cbz groups) and can then be labeled by removing the protective group of the initiator before coupling it to the dye. Depending on the nature of the blocking group, the removal of the protective group can be carried out by standard methods such as by TFA for BOC, piperidine in DMF for FMOC and by HBr for Cbz groups.

According to another aspect, the polymers of the invention are then obtained in an unblocked form (R¹ being for example maleimide, NHS ester, a hydrazide group, a hydrogen atom or an amine group).

For example, a biocompatible dye can be coupled to the amino group of the polymer, preferably under non-oxidative atmosphere, as described in Greg T. Hermanson, Bioconjugate Techniques, 3rd Edition (2013*)* or as described below. Before starting the deprotection steps, solvents can be removed from the polymer preparation by *vacuum* drying or freeze drying.

One advantage of polymers of the invention is to offer the possibility to covalently link targeting molecules that recognize specific target biomarkers to the polymer through a fast and simple so-called "click reaction" as described in Uttamapinant et al., 2012, Angew. Chem. Int. Ed. Engl., 51(24): 5852-5856*;* Hong et al., 2009, Angew. Chem. Int. Ed. Engl., 48(52): 9879-83*;* Binder and Sachsenhofer, 2007, Macromol. Rapid Commun., 28, 15-54 to the azide moiety on the azide bearing methacrylate monomer units, in particular Huisgen copper(I)-catalyzed azide-alkyne 1,3-dipolar cycloaddition (CuAAC), in presence of a stabilizing ligand, such as TBTA (tributylin acrylate) or THPTA (3[tris(3-hydroxypropyltriazolylmethyl)amine) or BTTAA (bis[(tertbutyl triazoyl)methyl]-[(2-carboxymethyltriazoyl)methyl]-amine). An advantage of this reaction for biological purposes is that the azide and alkyne functional groups are largely inert towards biological molecules and aqueous environments. For this purpose, targeting molecules that recognize specific target biomarkers are used as alkyne-bearing molecules.

Alkynation of the targeting molecules can be carried out by standard alkynation procedure. According to a specific embodiment, peptidic targeting molecules are prepared by adding a propargylglycine amino acid at the terminus of the peptide (for example C-terminus) as described, for example, in Jagasia et al., 2009, J. Org. Chem. 74(8): 2964-2974 to provide an alkyne moiety necessary for the click reaction with the azide moiety on the azide bearing methacrylate monomer units.

In another further aspect of the invention, is provided a method of preparation of a diagnosis probe comprising a step of coupling at least one of the azide groups of a block copolymer of the invention to a targeting molecule by azide-alkyne Huisgen cycloaddition. According to a further aspect, is provided a method of preparation of a diagnosis probe comprising a step of coupling a block copolymer of ω-azido oligoethyleneglycol methacrylate (AOEGMA), 3-sulfopropyl methacrylate (SPMA) and optionally hydroxyethyl methacrylate (HEMA) with at least one targeting molecule, for example by coupling at least one of the azide groups of the monomer AOEGMA to said targeting molecule, for example by azide-alkyne Huisgen cycloaddition.

According to a particular aspect, is provided a method of preparation of a diagnosis probe comprising the steps of:
(i) providing a block copolymer of the invention;
(ii) Reacting the said polymer with an alkyne bearing a targeting molecule under an azide-alkyne Huisgen cycloaddition;
(iii) Isolating the resulting polymer.

According to a particular aspect, is provided a method of preparation of a diagnosis probe, wherein the copolymer is of Formula (I).

According to a further particular embodiment, is provided a method of preparation of a diagnosis probe wherein the polymer provided under step (i) is a fluorescent polymer of Formula (I) (e.g. R¹ is a dye).

According to a further particular embodiment, is provided a method of preparation of a diagnosis probe wherein the polymer provided under step (i) is a fluorescent polymer of Formula (I').

According to a further particular embodiment, is provided a method of preparation of a diagnosis probe wherein the alkyne bearing targeting molecule is of Formula (III). According to another further particular embodiment, is provided a method of preparation of a diagnosis probe wherein the isolated polymer under step (iii) is of Formula (II): 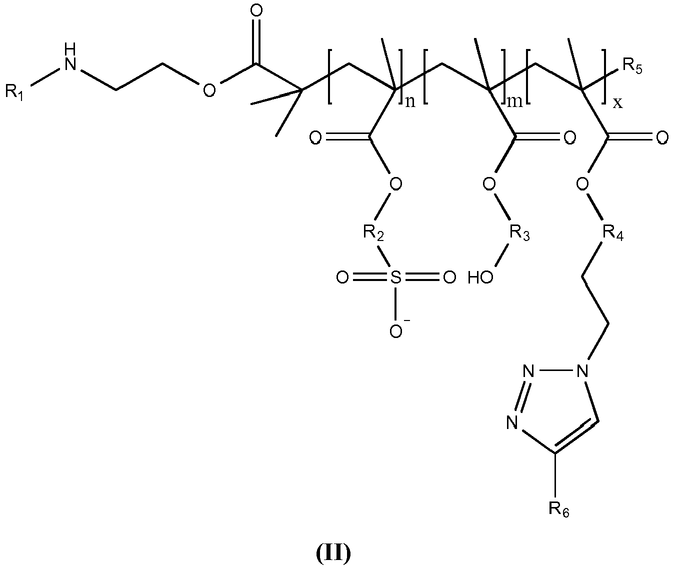 wherein m, n, x, R¹. R² R³, R⁴, R⁵ are as defined above and R⁶ is a targeting molecule.

According to another further particular embodiment, is provided a method of preparation of a diagnosis probe wherein the isolated polymer of Formula (II) is then labeled with a dye.

According to another further particular embodiment, is provided a method of preparation of a diagnosis probe wherein the isolated polymer of Formula (II), wherein m, n, x, R² R³, R⁴, R⁵ are as defined above, R¹ is selected from a blocking group, N-hydroxysuccinimide (NHS) ester, a maleimide group, a hydrazide group, a hydrogen atom, an amine group and a dye; R², R³ and R⁴ are independently selected from (CH₂)ₐ, benzyl and (CH₂CH₂O)_{b}, wherein a is an integer from 1 and 6, b is an integer from 1 and 8; and R⁶ is a targeting molecule.

According to another further particular embodiment, is provided a method of preparation of a diagnosis probe wherein the isolated polymer under step (iii) is of Formula (II') as represented on Figure 3C1-C2

According to a further particular aspect, is provided a method of preparation of a diagnosis probe according to the invention wherein the alkyne bearing targeting molecule is a peptide comprising a propargylglycine amino acid, for example at the C or N-terminus as described, for example, in Trost and Weiss, 2009, Adv. Synth. Catal. 351(7-8*).*

According to a further particular aspect, is provided a method of preparation of a diagnosis probe according to the invention wherein the alkyne bearing targeting molecule is a peptide of SEQ ID NO: 1 bearing an alkyne group either in the C-terminus or N-terminus of the sequence such as for example forming a molecule of Formula (III).

According to a further particular aspect, is provided a method of preparation of a diagnosis probe according to the invention wherein the alkyne bearing targeting molecule is a modified peptide of Formula (III).

According to a particular aspect, is provided a diagnosis probe comprising a targeting molecule of SEQ ID NO: 1 or a fragment or variant thereof and which is useful in targeting the inflamed tissue in a subject prone to colitis.

The diagnosis probes obtained by a method according to the invention can be purified by standard methods and combined to pharmaceutically acceptable agents for preparing diagnosis compositions such as described in the Handbook of Pharmaceutical Excipients, 6th Ed. Edited by Raymond C Rowe.

### Compositions

The invention provides polymers, pharmaceutical compositions thereof, and methods for diagnosing a disease or disorder and methods of treating a patient, preferably a mammalian patient, and most preferably a human patient that is suffering from or at high risk of suffering from gynecological and/or urological diseases or disorders such as a gynecological and/or urological cancer (typically a cervical or a prostate cancer) or such as a gynecological and/or urological infection (typically a yeast or fungi infection). The polymers of the polymers, pharmaceutical compositions thereof, and methods for diagnosing a disease or disorder and methods according to the invention are also useful for patients that are suffering from or at high risk of suffering from respiratory disorders such as lung fibrosis or lung infections.

The polymers of the polymers, pharmaceutical compositions thereof, and methods for diagnosing a disease or disorder and methods according to the invention are also useful for patients that are suffering from or at high risk of suffering from ocular disorders such as autoimmune macular degeneration.

The polymers of the polymers, pharmaceutical compositions thereof, and methods for diagnosing a disease or disorder and methods of treatment are particularly useful to for patients that are suffering from or at high risk of suffering from a hypersensitivity to an inflammatory bowel disease or a risk of developing inflammatory bowel disease, in particular Crohn's disease or ulcerative colitis (UC).

The polymers, pharmaceutical compositions thereof, and methods for diagnosing a disease or disorder and methods of treatment according to the invention are particularly useful to for patients that are suffering from or at high risk of suffering from a cancer, in particular gastrointestinal neoplastic disorders, in particular Barrett's oesophagus, oesophageal cancer, gastric cancer or colorectal cancer.

In a particular embodiment, the invention provides a copolymer of Formula (I) and a pharmaceutical formulation thereof wherein R¹ is a PDT dye for use as a medicament. In a further particular embodiment, the invention provides a copolymer of Formula (I) and a pharmaceutical formulation thereof wherein R¹ is a PDT dye useful as a theranostic agent or in theranostic therapy.

Pharmaceutical compositions of the invention can contain at least thereof according to the invention in any form described herein. Compositions of this invention may further comprise one or more pharmaceutically acceptable additional ingredient(s) such as alum, stabilizers, antimicrobial agents, buffers, coloring agents, flavoring agents, adjuvants, and the like.

The compositions according to the invention, together with a conventionally employed adjuvant, carrier, diluent or excipient may be placed into the form of pharmaceutical compositions, namely microparticulate systems and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral (including subcutaneous) use by injection or continuous infusion. Injectable compositions are typically based upon injectable sterile saline or phosphate-buffered saline or other injectable carriers known in the art. Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed. According to a particular embodiment, compositions according to the invention are injectable.

According to another particular embodiment, compositions according to the invention are oral formulations.

According to another particular embodiment, compositions according to the invention are oral formulations for the early detection, diagnosis or prevention and/or treatment of disease or disorder of inflammatory bowel diseases (IBDs) or colorectal cancer. According to another particular embodiment, compositions according to the invention are intra-ocular formulations.

According to another particular embodiment, compositions according to the invention are intra-vaginal formulations.

According to another particular embodiment, compositions according to the invention are intra-nasal formulations.

According to another particular embodiment, compositions according to the invention are intra-vesical formulations.

Compositions of this invention may be liquid formulations including, but not limited to, aqueous or oily suspensions, solutions, emulsions, syrups, and elixirs. The compositions may also be formulated as a dry product for reconstitution with water or other suitable vehicle before use. Such liquid preparations may contain additives including, but not limited to, suspending agents, emulsifying agents, non-aqueous vehicles, preservatives, dispersing or wetting agents and cryoprotectants. Suspending agents include, but are not limited to, sorbitol syrup, methyl cellulose, glucose/sugar syrup, gelatin, hydroxyethyl cellulose, carboxymethyl cellulose, aluminum stearate gel, and hydrogenated edible fats. Emulsifying agents include, but are not limited to, lecithin, sorbitan monooleate, and acacia. Preservatives include, but are not limited to, methyl or propyl p-hydroxybenzoate, ascorbic acid and sorbic acid. Dispersing or wetting agents include but are not limited to poly(ethylene glycol), glycerol, bovine serum albumin, Tween®, Span® or polyvinyl alcohol. Cryoprotectant agents include, but are not limited to, sucrose, trehalose, glucose, sorbitol, mannose or maltose.

Solid compositions of this invention may be in the form of tablets or lozenges formulated in a conventional manner. For example, tablets and capsules for oral administration may contain conventional excipients including, but not limited to, binding agents, fillers, lubricants, disintegrants and wetting agents. Binding agents include, but are not limited to, syrup, accacia, gelatin, sorbitol, tragacanth, mucilage of starch and polyvinylpyrrolidone. Fillers include, but are not limited to sugar, microcrystalline cellulose, maizestarch, calcium phosphate, and sorbitol. Lubricants include, but are not limited to, magnesium stearate, stearic acid, talc, polyethylene glycol, and silica. Disintegrants include, but are not limited to, potato starch and sodium starch glycolate. Wetting agents include, but are not limited to, sodium lauryl sulfate. Tablets may be coated according to methods well known in the art.

Compositions of this invention may also be formulated for inhalation, which may be in a form including, but not limited to, a solution, suspension, or emulsion that may be administered as a dry powder or in the form of an aerosol using a propellant.

In certain embodiments, the therapeutic compound(s) are directly administered as a pressurized aerosol or nebulized formulation to the patient's lungs via inhalation. Such formulations may contain any of a variety of known aerosol propellants useful for endopulmonary and/or intranasal inhalation administration. In addition, water may be present, with or without any of a variety of cosolvents, surfactants, stabilizers (e.g., antioxidants, chelating agents, inert gases and buffers). For compositions to be administered from multiple dose containers, antimicrobial agents are typically added. Such compositions are also generally filtered and sterilized, and may be lyophilized to provide enhanced stability and to improve solubility.

The pharmaceutical composition of the invention may consist of dosage units that can be administered as an aerosol. The term aerosol is used to denote a variety of systems ranging from those of colloidal nature to systems consisting of pressurized packages. Delivery may be by a liquefied or compressed gas or by a suitable pump system that dispenses the active ingredients. Aerosols of compounds of the invention may be delivered in single phase, bi-phasic, or tri-phasic systems in order to deliver the active ingredient(s). Delivery of the aerosol includes the necessary container, activators, valves, subcontainers, and the like, which together may form a kit. One of ordinary skill in the art, without undue experimentation may determine preferred aerosols. Compositions of this invention may also be formulated as a rectal delivery system such as described in Aulton 's "Pharmaceutics: The Design and Manufacture of Medicines", 4th Edition, 2013, Churchill Livingstone Elsevier*.*

Compositions of this invention may also be formulated as a vaginal delivery system such as described in *Aulton's "Pharmaceutics, 2013, supra.*

Further materials as well as formulation processing techniques and the like are set out in Part 5 of Part 5 of Remington's "The Science and Practice of Pharmacy", 22nd Edition, 2012, University of the Sciences in Philadelphia, Lippincott Williams & Wilkins the content of which is incorporated herein by reference.

### Mode of administration

Polymers and compositions of this invention may be administered in any manner including intravenous injection, intraperitoneal injection, subcutaneous injection, oral route, intranasal administration, intrapulmonary instillation or by inhalation, intraocular route, intravaginal, intrarectal or intravesical route. In certain embodiments, a combination of different routes may also be used.

The dosage administered, as single or multiple doses, to an individual will vary depending upon a variety of factors, including pharmacokinetic properties, patient conditions and characteristics (sex, age, body weight, health, size), extent of symptoms, concurrent treatments, site of diagnosis, frequency of treatment and the effect desired.

The polymers of the invention can be useful in many fields of diagnostics, besides in the diagnosis of gastrointestinal disorders.

According to a particular aspect, polymers of the invention can be useful in gynecological/urological applications, for example for gynecological/urological cancer diagnosis (e.g. cervix, prostate etc..) or gynecological/urological infections (e.g. by yeast, fungi etc...). In this case, the targeting molecules that recognize specific target biomarkers that will be used will be specific to the gynecological/urological cancer or gynecological/urological infection, for examples such as procalcitonin (PCT), mammalian target of rapamycin (mTOR), poly-ADP-ribose polymerase (PARP) and components of the EGFR pathway.

According to another particular aspect, polymers of the invention can be useful in respiratory tract applications such as for the diagnosis of lung affections (e.g. pulmonary fibrosis, lung infections, lung cancer etc...). In this case, the targeting molecule that recognizes a specific target biomarker that will be used will be specific to mammalian target of rapamycin (mTOR) and RAS / PI3K involving pathways. According to another particular aspect, polymers of the invention can be useful in ocular applications such as for example, for the diagnosis of ocular disorders such as autoimmune macular degeneration. In this case, the targeting molecule that recognizes a specific target biomarker that will be used will be specific to ocular disorders such as described in Ross et al., Expert Rev Ophthalmol., 2007, 2(3): 443-457*.*

### Combination

According to the invention, polymers of the invention and pharmaceutical formulations thereof can be administered alone or in combination with a co-agent useful in the diagnostic procedure.

Possible co-agents further include colonic preparations for bowel cleansing prior colonoscopy for example, such as described in Park and Lim, World J Gastroenterol. 2014 March 21; 20(11): 2735-2740.

According to one aspect, the invention encompasses the administration of a polymer of the invention a pharmaceutical formulations thereof to an individual prior to, simultaneously or sequentially with other therapeutic/prophylactic regimens or co-agents in the prevention, diagnosis or treatment of particular Inflammatory Bowel Disease, in particular Crohn's disease and ulcerative colitis (e.g. combined drug regimen), or of coeliac disease in a therapeutically effective amount. A polymer of the invention, or the pharmaceutical formulation thereof that is administered simultaneously with said co-agents can be administered in the same or different composition(s) and by the same or different route(s) of administration.

According to one embodiment, is provided a pharmaceutical formulation comprising at least one polymer of the invention combined with at least one co-agent useful in the prevention and/or treatment of Inflammatory Bowel Disease in particular Crohn's disease and ulcerative colitis, and coeliac disease, at least one pharmaceutically acceptable carrier, diluent or excipient thereof.

### Patients

In an embodiment, patients according to the invention are patients suffering from an Inflammatory Bowel Disease disorder such as Crohn's disease, ulcerative colitis, lymphocytic colitis, ischemic colitis, Bechet's disease, diversion colitis, and irritable bowel syndrome, or coeliac disease.

In another embodiment, patients according to the invention are patients at risk of suffering from Crohn's disease.

In another further embodiment, patients according to the invention are suffering from colitis such as lymphocytic colitis, ischemic colitis, diversion colitis and ulcerative colitis.

In another further embodiment, patients according to the invention are suffering from ulcerative colitis.

In another further embodiment, patients according to the invention are suffering from coeliac disease or at risk of suffering from coeliac disease due to family history.

In another embodiment, patients according to the invention are patients at risk of suffering from hypersensitivity/irritability of the colon and/or small intestine.

In another embodiment, patients according to the invention are patients suffering from a gastrointestinal neoplastic disorder such as Barrett's oesophagus, oesophageal cancer, gastric cancer or colorectal cancer.

In another further embodiment, patients according to the invention are suffering from colorectal cancer.

In another embodiment, patients according to the invention are patients suffering from or are at risk of suffering from a gynecological/urological cancer or gynecological/urological infections (e.g. by yeast, fungi etc...).

In another embodiment, patients according to the invention are patients suffering from or are at risk of suffering from lung affections.

In another embodiment, patients according to the invention are patients suffering from or are at risk of suffering from ocular disorders such as age-related wet macular degeneration.

### Use according to the invention

In accordance with one aspect of the present invention, is provided a use of a block copolymer for the preparation of a medical composition for the diagnosis or prevention and/or treatment of disease or disorder, and in particular of inflammatory bowel diseases (IBDs), in particular Crohn's disease (CD) and ulcerative colitis (UC) as well as coeliac disease and cancers such as a colorectal cancer.

According to a further particular aspect, is provided a use of block copolymer of the invention for the preparation of a medical composition for the diagnosis or prevention and/or treatment of disease or disorder, such as those described herein.

In accordance with one aspect of the present invention, there is provided a method for detecting, preventing, repressing or treating a disorder or a disease in a subject, said method comprising administering in a subject in need thereof or contacting a tissue in a said subject with an effective amount of at least one di- or tri-block copolymer of the invention a or a pharmaceutical formulation thereof.

According to a particular aspect, the method is a method of detecting a disease or disorder, in particular IBDs, in particular Crohn's disease (CD) and ulcerative colitis (UC), as well as coeliac disease or a cancer such as a colorectal cancer wherein the polymer of the invention is a diagnosis probe according to the invention comprising at least one targeting molecule that recognizes a specific biomarker for those disorders. According to a particular aspect, the method is a method of detecting and treating a disease or disorder (theranostics), wherein the polymer of the invention is a diagnosis probe according to the invention comprising at least one targeting molecule that recognizes a specific biomarker for those disorders and an agent able to eliminate the undesired cells or tissue (e.g. by using photosensitizers, photodynamic therapy).

In a particular aspect, a method of detecting a disease or disorder according to the invention in a subject comprises the steps of:
(i) *Ex-vivo* contacting a biological sample or a tissue with, or administering in a subject in need thereof, a diagnosis probe comprising a block copolymer of the invention comprising a targeting molecule specific of said disease and disorder and a biocompatible dye;
(ii) Detecting the specific binding of the diagnosis probe;
(iii) Comparing the said specific binding to a reference (for example to the binding of said diagnosis probe in a reference or control sample).

According to a particular aspect, an increase in the specific binding detected under step (ii) as compared to a reference is indicative of the onset or progression of said disease or disorder.

The control sample is a biological sample that can be from the same subject, a different subject, or it can be a pooled sample from a number of disease-free subjects. According to a particular embodiment, the control sample is a non-diseased or inflamed tissue of the subject.

According to a particular aspect, a method of detecting a disease or disorder according to the invention is an *ex vivo* method.

According to a particular aspect, is provided a method of detecting a disease or disorder according to the invention wherein the diagnosis probe is a diagnosis probe of Formula (II), wherein m, n, x, R¹, R², R³, R⁴ and R⁵ are as defined above and R⁶ is a targeting molecule.

According to another particular aspect, is provided a diagnosis probe obtainable by a method according to the invention.

According to another particular aspect is provided a diagnosis probe according to Formula II'.

According to another particular aspect is provided a alkyne bearing targeting molecule according to Formula III.

In one embodiment of the invention is provided a use of a polymer or a formulation thereof according to the invention for the preparation of a pharmaceutical composition for the prevention, repression and/or treatment of a disease or disorder, in particular Inflammatory Bowel Disease disorder, in particular Crohn's disease or ulcerative colitis.

In another embodiment of the invention is provided a use of a polymer or a formulation thereof according to the invention for the preparation of a pharmaceutical composition for the prevention, repression and/or treatment of a cancer, in particular gastrointestinal neoplastic disorders, in particular Barrett's oesophagus, oesophageal cancer, gastric cancer or colorectal cancer.

In a further embodiment of the invention is provided a use or a method according to the invention, wherein the subject is predisposed or at risk to develop hypersensitivity/irritability of the colon and/or small intestine, for example based on familial history, personal history, any of the symptoms listed above, or age.

In another embodiment, is provided a polypeptide, a composition or a method according to the invention wherein composition thereof is to be administered by the oral, intranasal, intraperitoneal, intravaginal, intravesical, intraocular, parenteral or systemic route.

In another embodiment, is provided a medicinal kit comprising a diagnosis probe according to the invention according to the invention together with instructions of use. Examples illustrating the invention will be described hereinafter in a more detailed manner and by reference to the embodiments represented in the Figures.

### EXAMPLES

The following abbreviations refer respectively to the definitions below:
**ATRP** (atom transfer radical polymerization); **DMSO** (Dimethyl sulfoxide); **DSS** (dextran sodium sulfate); **EDTA** (Ethylene diamine tetraacetic acid); **IBD** (Inflammatory Bowel Disease); **PBS** (Phosphate Buffered Saline); **TFA** (tri-fluoro acetic acid).

### Example 1: Synthesis of polymers according to the invention

A polymer of the invention, in particular a copolymer of Formula (I), wherein R¹ is BOC, R² is (CH₂)₃, R³ is (CH₂)₂, R⁴ is (CH₂)₂O)₂, R⁵ is Br (copolymer of Formula (Ia)) is synthesized by atom transfer radical polymerization (ATRP) in presence of an alkyl halide initiator as shown below under Scheme 4. Two of the monomers employed in the polymerization reaction were commercially available (SPMA and HEMA), the AOEGMA monomer and the alkyl halide initiator were synthesized as shown under Schemes 3 and 2, respectively.

### Alkyl halide initiator synthesis

To synthesize an alkyl halide initiator of Formula (ii), wherein R¹ is BOC and R⁵ is Br (Formula (iia)), BOC-ethanolamine (ia) (1 ml, 6.45 mmol) and pyridine (1.565 ml, 19.35 mmol) were mixed with 10 mL methylene chloride in a septum covered 50 mL round-bottomed flask with two overtures (in an ice bath) and a magnetic stirrer. Bromoisobutyryl bromide (i'a) (1.171 ml, 7.74 mmol) was added dropwise through a needle during 10-15 min as shown under Scheme 2 below. N₂ needle was placed in the septum to avoid overpressure. The reaction product was purified by silica gel column to obtain BOC-aminoethyl 2-bromo-2-methylpropionate (BABMP) initiator (alkyl halide initiator) of Formula (iia) and freeze dried.

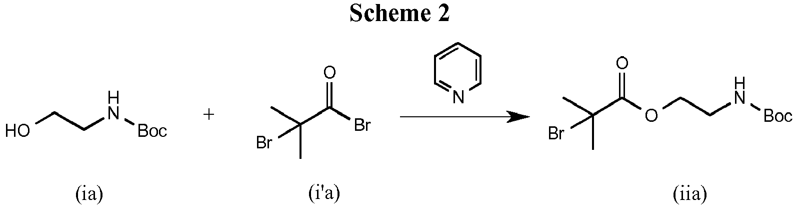

### Synthesis of AOEGMA monomer

For the synthesis of the AOEGMA monomer (azide bearing monomer according to Formula (M3) wherein R⁴ is (CH₂CH₂O)₂), sodium azide (4 g, 61.5 mmol) and potassium iodide (1 mg, 6.02 µmol) were added to a 30 mL Milli-Q™ water solution containing triethylene glycol monochloridine (Sigma) (4.31 ml, 29.7 mmol). Reaction was stirred in a reflux condenser at 80°C for 12 h. Reaction mixture was extracted with chloroform three times and organic layers were dried on anhydrous sodium sulphate and evaporated to give a transparent liquid corresponding to azide triethylene glycol as shown under Scheme 3 below. Freshly distilled methacryloyl chloride (Alfa Aesar)

(8.49 ml, 87 mmol) was added dropwise to a solution of the azide triethylene glycol obtained above (4 g, 22.83 mmol) and triethylamine (12.18 ml, 87 mmol) in 30 mL of methylene chloride in an ice bath at 4°C. The mixture is allowed to react under stirring at 4°C for 12 h. The reaction product was then filtered, concentrated and purified through silica column to obtain a light, yellow oil.

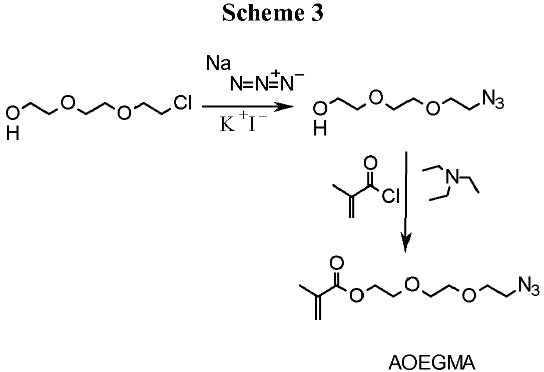

### Synthesis of the polymer

A polymer of Formula (I), wherein R¹ is BOC, R² is (CH₂)₃, R³ is (CH₂)₂, R⁴ is (CH₂CH₂O)₂ and R⁵ is Br (Formula (Ia)), was synthesized by ATRP under the reaction depicted under Scheme 4 and using the starting materials listed under Table 1 below (The letters a, b and c represent the different composition of the polymer):

**Table 2**

| ***Starting material*** | ***MW*** | ***Amount (mg)*** | ***n*** | *Eq* |
|---|---|---|---|---|
| Alkyl halide initiator (BABMP) (iia) | 310.188 | 2.5 | 8.060 µmol | 1 |
| AOEGMA (M3 wherein R⁴ is (CH₂CH₂O)₂) | 243.263 | | | a |
| SPMA Sulfopropyl methacrylate (SIGMA) (M1 wherein R² is (CH₂)₃) | 246.318 | | | b |
| HEMA Hydroxyethyl methacrylate (SIGMA) (M2 wherein R³ is (CH₂)₂) | 130.143 | | | c |
| 2,2-Bipyridine (SIGMA) | 156.188 | 18.88 | 0.121 mmol | 15 |
| Copper Bromide (I) (SIGMA) | 143.450 | 5.78 | 0.040 mmol | 5 |

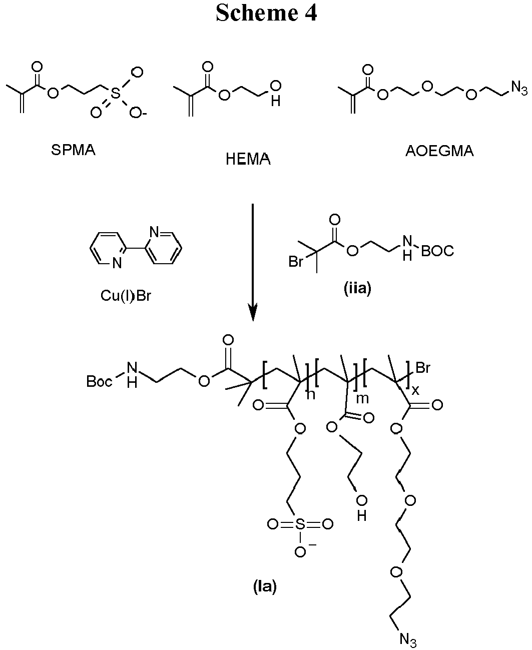

In a septum covered round-bottomed flask (50-100 mL) with a magnetic stirrer, 2,2'-bipyridine (i) (18.9 mg, 0.121 mmol), copper (I) bromide (5.78 mg, 0.040 mmol), SPMA and distilled HEMA are dissolved in 12 mL Milli-Q™ water. While degassing with argon, 17 mL of methanol are added to the reaction mixture. The initiator (iia) (2.5 mg, 0.008 mmol) and AOEGMA previously dissolved in 1 mL of methanol are finally added to the flask. Everything is dissolved completely and the mixture is degassed for 10 min. Put into the oil bath at 45°C and let it react for 24 h. To stop the reaction, open the septum and let air come in. The reaction mixture should turn from a dark-brown color to blue, indicating aerial oxidation of Cu(I) to Cu(II). After the polymerization, the product is concentrated in the rotavap (Buchi™ Rotavapor R-210) as much as possible and dialyzed (SpectraPor™ Regenerated Cellulose membranes, MWCO: 6-8000 Da) against water with EDTA for 48 h to remove the unreacted monomers and copper. Then dialyze against water for another 48 h. The polymer is then recovered by freeze-drying.

The polymer was then characterized in terms of monomer composition and molecular weight (MW) by 1H-NMR and FTIR spectroscopy and size exclusion chromatography (SEC).

NMR spectra were recorded on a Bruker Av400 spectrometer (Bruker BioSpin, Fällanden, Switzerland) operating at 400 MHz for protons. Polymer samples (5 mg) are dissolved in 0.5 mL deuterium oxide at 25°C. The polymer composition was determined from the ratio intensity of the methyl peak (2.8-3.0 ppm) of SPMA to that of the methyl peaks of HEMA (3.7-4.1 ppm) and AOEGMA (3.4-3.5 ppm). Data is analyzed by MestReNova v.6.2.1 software.

FTIR was used to qualitatively detect the presence/absence of the azide groups in the polymer structure. Spectra were obtained using ATR geometry on a Spectrum 65 infrared spectrometer (Perkin-Elmer, Schwerzenbach, Switzerland). A sample of the freeze dried product (1 mg) was loaded into the spectrometer plate and the transmittance was measured. Data is analyzed by Spectrum Software.

The polymer molecular weight was determined by SEC with a Viscotek TDAmax system (Viscotek, Houston, TX) equipped with refractive index and light scattering detectors. Two mixed bed aqueous SEC column (TSKgel™ GMPWxl, 7.8mm x 30cm, hydroxylated polymethacrylate, Viscotek) are used in series to achieve good separation. Aqueous sodium azide (0.3 % w/v, Sigma) is used as eluent with a flow rate of 0.6 mL/min. Average molecular weights are given relative to polyethylene oxide (PEO) and dextran standards (Malvern, UK). Data is analyzed by OmniSEC v.4.5 software.

The PDI was calculated/measured by the ratio Mw/Mn, being Mn the number average molecular weight (statistical average molecular weight of all the polymer chains in the sample) and Mw the weight average molecular weight (compared to Mn, Mw takes into account the molecular weight of a chain in determining contributions to the molecular weight average. The more massive the chain, the more the chain contributes to Mw).

Examples of polymers according to the invention synthesized according to the above procedure are presented in Table 3 below:

**Table 3**

| ***Polymers*** | ***Theoretical Composition (%) SPMA:HEMA:AOEGMA*** | ***Obtained Composition (%) SPMA:HEMA:AOEGMA*** | ***MW (PDI)*** |
|---|---|---|---|
| Poly-A | 10:85:5 | 6:90:4 | 51.9 kDa (1.2) |
| Poly-B | 20:75:5 | 14:81:5 | 55.6 kDa (1.5) |
| Poly-C | 50:45:5 | 39:58:3 | 50.8 kDa (1.8) |
| Poly-D | 70:25:5 | 63:34:3 | 53.2 kDa (1.9) |
| Poly-E | 70:20:10 | 68:23:9 | 89.0 kDa (1.5) |
| Poly-F | 80:15:5 | 77:20:3 | 59.1 kDa (1.7) |
| Poly-G | 90:5:5 | 91:6:3 | 63.8 kDa (1.3) |
| Poly-H | 95:0:5 | 97:0:3 | 58.6 kDa (1.3) |

| | | | |
|---|---|---|---|
| MW: Molecular Weight; PDI: Polydispersity Index | | | |

### Example 2: Polymer stability

The polymer stability was tested in simulated gastric and intestinal fluids at 37°C by gel permeation chromatography (GPC) as described in United States Pharmacopeia 33-28NF (2010) and European Pharmacopeia 7.0 (2010). Polymers were incubated with gastrointestinal fluids for 2 h. Enzymes were then heat-inactivated at 95°C for 10 min, centrifuged, filtered through 0.2 µm acetate cellulose filters and injected in the GPC analyser. None of the polymers showed any sign of degradation when incubated in simulated gastric fluid (SGF) and simulated intestinal fluid (SIF) over 2 hours since the structure of the polymers is maintained and no change in retention volume is observed after incubation.

### Example 3: Polymer interaction with the GI tract

The interaction of the polymer with the mucins present on the GI tract was tested in vitro as described in Fuhrmann et al., 2013, Nature Chemistry, 5, 582-589*,* with slight modifications. Mucin from porcine stomach, Type II, (Sigma) was dissolved in PBS at a concentration of 3 mg/mL. Mucin supernatant at a final concentration of 1 mg/mL is incubated with polymer solutions for 30 min at 37°C at different polymer/mucin ratios (w/w) (0, 0.25, 0.5, 0.75 and 1) and different pH (1.2, 4.5, 6.8 and 7.4). Poly-D-Lysine

(Sigma) was used as a control. The absorbance at 400 nm was measured. Very low or no interaction of the polymer with the mucin was observed.

### Example 4: Labeling the polymer with a flurorescent probe

The labeling of a polymer of Formula (Ia) with a dye is depicted in Scheme 5 below. The protective group (e.g. BOC group) of polymers of Formula (Ia) (10 mg) is removed by incubation with 4 mL TFA for three hours under magnetic stirring at room temperature. Then, the TFA-polymer mixture is diluted with 50 mL toluene in order to evaporate the solvent under reduced pressure. In order for the fluorescent dye coupling reaction to take place, the amino group that will further react with the dye should not be protonated, therefore, polymers are then neutralized with NaOH 1 M and dialysed against water overnight to remove the residual TFA and salts. Deprotected polymers (Formula (Ib)) are then freeze dried. For the coupling of the dye to the deprotected polymer, inert gas atmosphere is employed at all times in order to avoid oxidation of the dye. The polymer (100 nmol) is dissolved in 200 µL of anhydrous DMSO. The monofunctional activated polymer is labeled in the dark at room temperature under magnetic stirring for 4 h with an excess of biocompatible fluorescent dye N-hydroxysuccinimide ester (DyLight™ 680 NHS (Pierce Technologies)) (300 nmol) predissolved in anhydrous DMSO. The crude reaction is then diluted in Milli-Q™ water and the unreacted dye is removed by Amicon™ Ultra diafiltration (e.g. MWCO 10 kDa, 4500 g, 30 min). The dye-bound polymer of Formula (Ic) is then freeze dried.

The successfulness of the deprotection reaction can be assessed by the disappearance of the BOC peak at 1.4 ppm by NMR spectroscopy. The efficiency of the coupling procedure is assessed spectrophotometrically in water on a Tecan Infinite® 200 PRO series microplate reader, depending on the spectrophotometric characteristics of the dye. After the dye coupling reaction and purification by Amicon™ Ultra centrifugal devices, 5 µL of sample are taken and diluted with 95 µL water in a 96-well plate to measure the absorbance at the excitation wavelength of the dye. The rest of the product is freeze dried to measure the net material weight that was recovered. A calibration curve with the free dye is also performed to back-calculate the amount of dye coupled to the polymer from the absorbance measured.

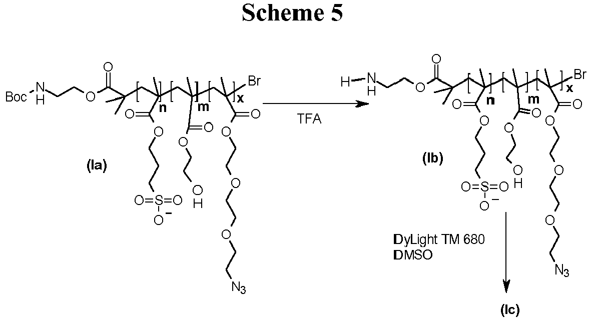

### Example 5: Coupling targeting molecules to the flurorescent dye-polymer construct

One or more copies of the same targeting molecule that recognizes a specific target biomarker or of different targeting molecules that recognize specific target biomarkers can be coupled to the fluorescent dye-polymer construct through an azide-alkyne Huisgen cycloaddition ("click" chemistry) using a water-soluble ligand for Cu^{I} such as THPTA and an alkyne-bearing targeting molecule as described under Scheme 6 below to lead to a compound of Formula (II') wherein R¹ is a dye described in Example 4, R² is (CH₂)₃, R³ is (CH₂)₂, R⁴ is ((CH₂)₂O)₂ and m, n, x, R⁵ and R⁶ are as defined above (compound of Formula (II')).

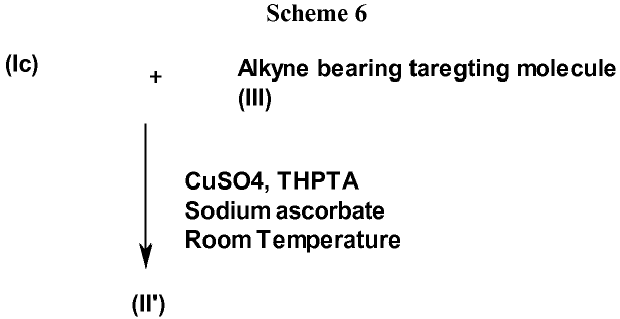

Table 4 below lists the starting materials used for this reaction.

**Table 4**

| ***Starting material*** | ***MW (Da)*** | ***Amount*** |
|---|---|---|
| THPTA ligand (Base Click) | 434.50 | 0.4 µmol |
| Alkyne-bearing targeting molecule (Formula (III)) | | |
| Fluorescent polymer (Formula (I)) | | |
| Copper sulfate (SIGMA) | 159.62 | 1.2 µmol |
| Sodium Ascorbate (SIGMA) | 198 | Excess |

First, 0.4 µmoL of THPTA ligand are mixed with 1.2 µmoL of copper sulfate for 5 minutes. A light blue solution is obtained. In the meantime, the polymer is dissolved in 200 µL of Milli-Q™ water. An alkyne-bearing targeting molecule (prepared by means of the addition of a propargylglycine aminoacid at the C-terminus) is added and the preparation is mixed completely. Then, the previously prepared ligand-Cu mixture is added and the preparation is mixed again. Finally, the sodium ascorbate is added to reduce Cu^{(II)} to Cu^{(I)}. Argon is filled into the eppendorf before closing it to avoid the oxidizing environment. Everything is mixed completely and incubated overnight under magnetic stirring at room temperature. Sample is then diluted in 15 mL Milli-Q™ water, dialyzed against water for 24 h (SpectraPor™ Regenerated Cellulose membranes, MWCO: 6-8000 Da) and freeze dried.

The targeting molecule-coupled fluorescent-dye polymer of Formula (II) is then analyzed by FTIR, in order to follow the qualitative disappearance of the azide peak at 2100 cm⁻¹. A sample of the freeze dried product (1 mg) is loaded into the spectrometer plate and the transmittance is measured. Data is analyzed by Spectrum Software.

### Example 6: Preparation of a polymer of the invention

The following example of polymer of the invention has been prepared and coupled to a biocompatible NIR flurorescent dye and a peptidic targeting molecule that selectively binds to mouse inflamed tissue in order to support its efficacy in the recognition of mouse inflamed colon.

A tri-block polymer of Formula (I) with a final composition of 68:23:9 (SPMA to HEMA to AOEGMA) % weight ratios for a total molecular weight of 89 kDa, namely where n is 246, m is 157 and x is 33 was synthesized by atom transfer radical polymerization (ATRP) as described in Example 1. The NIR flurorescent dye DyLight™ 680 was coupled to the tri-block polymer as described in Example 2 and a peptidic targeting molecule was then coupled to azide groups of the AOEGMA monomers of the tri-block polymer as described in Example 3. For the synthesis of a tri-block polymer of Formula (I), the following starting materials were used:

**Table 5**

| ***Starting material*** | ***MW*** | ***Amount*** | ***N*** | ***Eq*** |
|---|---|---|---|---|
| Alkyl halide initiator (BABMP) | 310.188 | 2.5 mg | 8.060 µmol | 1 |
| AOEGMA | 243.263 | 75 µL | 0.322 mmol | 40 |
| Sulfopropyl methacrylate (SIGMA) | 246.318 | 794 mg | 3.220 mmol | 400 |
| Hydroxyethyl methacrylate (SIGMA) | 130.143 | 130 µL | 1.072 mmol | 133 |
| 2,2-Bipyridine (SIGMA) | 156.188 | 18.88 mg | 0.121 mmol | 15 |
| Copper Bromide (I) (SIGMA) | 143.450 | 5.78 mg | 0.040 mmol | 5 |

In a septum covered round-bottomed flask (50-100 mL) with a magnetic stirrer, 2,2'-bipyridine (18.9 mg, 0.121 mmol), copper (I) bromide (5.78 mg, 0.040 mmol), SPMA (794 mg, 3.22 mmol) and distilled HEMA (130 µL, 1.072 mmol) are dissolved in 12 mL Milli-Q™ water. While degassing with argon, 17 mL of methanol are added to the reaction mixture. The initiator (BABMP) (2.5 mg, 0.008 mmol) and AOEGMA (75 µL, 0.322 mmol) previously dissolved in 1 mL of methanol are finally added to the flask. Everything is dissolved completely and the mixture is degassed for 10 min. Put into the oil bath at 45°C and let it react for 24 h. To stop the reaction, open the septum and let air come in. The reaction mixture should turn from a dark-brown color to blue, indicating aerial oxidation of Cu(I) to Cu(II). After the polymerization, the product is concentrated in the rotavap (Buchi™ Rotavapor R-210) as much as possible and dialyzed (SpectraPor™ Regenerated Cellulose membranes, MWCO: 6-8000 Da) against water with EDTA for 48 h to remove the unreacted monomers and copper. Then, the product is dialyze against water for another 48 h. The polymer is then recovered by freeze-drying.

The polymer was characterized in terms of monomer composition and molecular weight (MW). Composition of the polymer was assessed by ¹H-NMR and FTIR. NMR spectra were recorded on a Bruker Av400 spectrometer (Bruker BioSpin, Fällanden, Switzerland) operating at 400 MHz for protons. Polymer samples (5 mg) were dissolved in 0.5 mL deuterium oxide at 25°C. The copolymer composition was determined from the ratio intensity of the methyl peak (2.8-3.0 ppm) of SPMA to that of the methyl peaks of HEMA (3.7-4.1 ppm) and AOEGMA (3.4-3.5 ppm) as shown on Figure 1. Data was analyzed by MestReNova v.6.2.1 software.

FTIR was used to qualitatively detect the presence of the azide groups in the polymer structure. Spectra were obtained using ATR geometry on a Spectrum 65 infrared spectrometer (Perkin-Elmer, Schwerzenbach, Switzerland) as shown on Figure 2. A sample of the freeze dried product (1 mg) was loaded into the spectrometer plate and the transmittance was measured. Data was analyzed by Spectrum Software.

The polymer MW was determined by size exclusion chromatography (SEC) with a Viscotek TDAmax system (Viscotek, Houston, TX) equipped with refractive index and light scattering detectors. Two mixed bed aqueous SEC column (TSKgel™ GMPWxl, 7.8mm x 30cm, hydroxylated polymethacrylate, Viscotek) were used in series to achieve good separation. Aqueous sodium azide (0.3 % w/v, Sigma) was used as eluent with a flow rate of 0.6 mL/min. Average molecular weights are given relative to polyethylene oxide (PEO) and dextran standards (Malvern, UK). Data was analyzed by OmniSEC v.4.5 software. Average number MW (Mn) of the polymer was 89 kDa. According to NMR and SEC data, the composition of the tri block polymer of the invention was calculated and Table 6 shows the calculation of the monomer composition of the polymer.

**Table 6**

| | Observed composition (NMR) % | | | | | | | |
|---|---|---|---|---|---|---|---|---|
| Polymer | SPMA: HEMA : AOEGMA | Polymer MW (GPC) (kDa) | | % | | MW (Da) | Mono mer MW (Da) | Monomer units |
| | | | SPMA | 68 | a | a=60520 | 246.3 | n=60520/246.3 = 246 |
| Poly-E | 68:23:9 | 89.0 | HEMA | 23 | b | b=20470 | 130.1 | m=20470/130.1 = 157 |
| | | | AOEGMA | 9 | c | c=8010 | 243.2 | x=8010/243.2 = 33 |

Then, the polymer of Formula (I) wherein n is 246, m is 157 and x is 33, R² is (CH₂)₃. R³ is (CH₂)₂., R⁴ is (CH₂CH₂O)_{2.}R⁵ is Br and R¹ is BOC (Poly-E) was coupled to the NIR dye DyLight™ 680 NHS ester as follows:
The protective BOC group (R¹) of the polymer (10 mg) is removed by incubation with 4 mL TFA for three hours under magnetic stirring at room temperature. Then, the TFA-polymer mixture is diluted with 50 mL toluene in order to evaporate the solvent under reduced pressure. In order for the dye coupling reaction to take place, the amino group that will further react with the dye should not be protonated; therefore, polymers are then neutralized with NaOH 1 M and dialysed against water overnight to remove the residual TFA. Deprotected polymers are then freeze dried. For the coupling of the NIR dye to the deprotected polymer, Ar atmosphere is employed at all times in order to avoid oxidation of the dye. The polymer (100 nmol) is dissolved in 200 µL of anhydrous DMSO. The monofunctional activated polymer is labelled in the dark at room temperature under magnetic stirring for 4 h with an excess of DyLight™ 680 NHS ester (28 µL, 300 nmol) predissolved in anhydrous DMSO (1 mg DyLight™/100 µL DMSO = 10.5 nmol/µL). The crude reaction is then diluted in Mili-Q water and the unreacted dye is removed by Amicon™ Ultra diafiltration (MWCO 10 kDa, 4500 g, 30 min). The dye-bound polymer is then freeze dried.

The successfulness of the deprotection reaction was assessed by the disappearance of the BOC peak at 1.4 ppm in NMR and the efficiency of the coupling procedure was assessed spectrophotometrically in water on a Tecan Infinite® 200 PRO series microplate reader. After the dye coupling reaction and purification by Amicon™ Ultra centrifugal devices, 5 µL of sample were taken and diluted with 95 µL water in a 96-well plate to measure the absorbance at the excitation wavelength of the dye (680 nm). The rest of the product was freeze dried to know the net material weight recovered. A calibration curve with the free dye was performed to back-calculate the amount of dye coupled to the polymer from the absorbance measured and it was determined that around 70 nmol of dye were bound to 76 nmol of polymer, which is 92% of binding efficiency, therefore the yield was 76 %. The resulting polymer is a polymer of Formula (I) wherein n is 246, m is 157 and x is 33 and R¹ is DyLight™ 680 is as follows (Formula (I')):

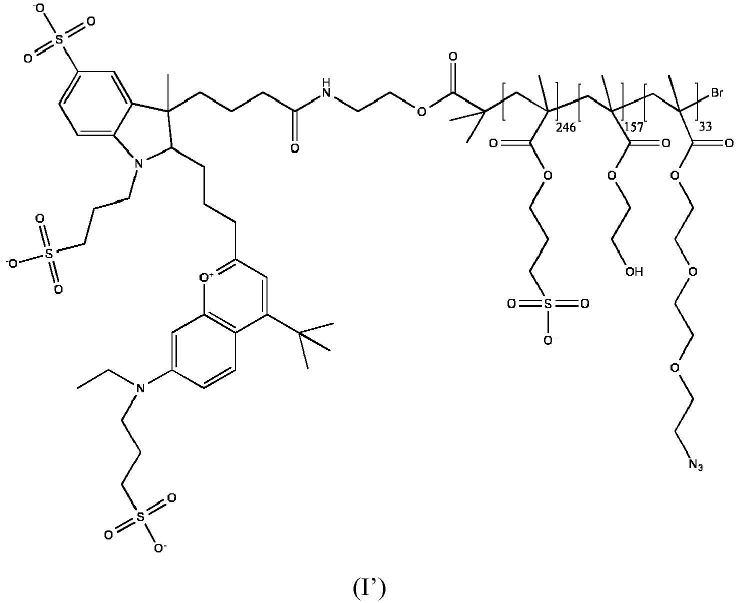

Then, the dye labeled polymer obtained above was coupled to an alkynated targeting molecule through an azide-alkyne Huisgen cycloaddition ("click" chemistry) as described below.

The targeting molecule is a peptide with the sequence SWTLYTPSGQSK of SEQ ID NO: 1 of the structure depicted on Figure 3A which corresponds to a peptide of a murine N-cadherin antagonist described in Devemy et al., 2008, Peptides 29, 1853-1861 which was described as an anti-angiogenic agent. Some authors have reported the structure of the human N-cadherin gene and described sequence homologies between human and mouse N-cadherin and other cadherins (Wallis et al., 1994, Genomics. 22(1) 172-179*).* In the current study, the peptide of SEQ ID NO:1 is used as targeting molecule for mouse inflamed colon since it is known that N-cadherin is overexpressed in IBD (Burke et al., 2011, Inflammatory Bowel Diseases, 17(8):1665-1673*).*

The peptidic targeting molecule is first alkynated in its C-term by the addition of a propargylglycine, an alkynated amino acid at the end of the sequence after a three-glycine linker to lead to an alkyne bearing targeting molecule of Formula (III) (Figure 3B). Table 7 shows the components used for the click reaction with the alkynated peptidic targeting molecule:

**Table 7**

| ***Starting Material*** | ***MW (Da)*** | ***Amount*** |
|---|---|---|
| THPTA ligand (Base Click) | 434.50 | 0.4 µmol |
| Alkyne bearing peptide (Formula III) | 1621 | 3.1 µmol |
| Polymer of Formula (I') | 89000 | 2.5 µmol azide |
| Copper sulfate (SIGMA) | 159.62 | 1.2 µmol |
| Sodium Ascorbate (SIGMA) | 198 | Excess |

The reagents were prepared as follows:
- Polymer: 6.87 mg (68:23:9; 89 kDa) = 76 nmol (x 33 azides/pol. unit = 2.5 µmol azide)
- Alkyne bearing peptide: Dissolve 5 mg in 333 µL → 3.1 µmol, 333 µL
- Copper sulfate (SIGMA) (Conc: 7.5 µmol/mL): For 1.2 µmol, 160 µL
- THPTA ligand (Base Click) (Conc: 9.2 µmol/mL): For 0.4 µmol, 40 µL
- Sodium ascorbate (SIGMA): 18 mg in 0.3 mL of water (conc: 240 µmol/mL). Add 200 µL.

First, 40 µL of THPTA ligand were mixed with 160 µL of copper sulfate for 5 minutes. A light blue solution was obtained. In the meantime, the dye labeled polymer containing the azide groups of the AOEGMA monomers was dissolved in 200 µL of Mili-Q water. 333 µL of Alkyne bearing pepeitde were added and everything was mixed completely. Then, the previously prepared ligand-Cu mixture was added and everything was mixed again. Some argon was put in the eppendorf before closing it to avoid the oxidizing environment. Finally, the 200 µL of Sodium Ascorbate were added to reduce Cu(II) to Cu(I). Everything was mixed completely and incubated overnight under magnetic stirring at room temperature. Sample was then diluted in 15 mL water, dialyzed against water for 24 h (SpectraPor Regenerated Cellulose membranes, MWCO: 6-8000 Da) and freeze dried.

The obtained polymer of the invention is of Formula (II) wherein n is 246, m is 157 and x is 33, R¹ is DyLight™ 680, R⁵ is Br and R⁶ is a peptide of SEQ ID NO: 1, R² is (CH₂)_{3.} R³ is (CH₂)_{2.} and R⁴ is (CH₂CH₂O)₂ (Formula (IIa)) was analyzed by FTIR, in order to see the qualitative disappearance of the azide peak at 2'100 cm-1 and the appearance of two broad peaks corresponding to amide bonds of the peptide at around 1'700 and 3'250 cm⁻¹ as shown on Figure 4. A sample of the freeze dried product (1 mg) was loaded into the spectrometer plate and the transmittance was measured. Data was analyzed by Spectrum Software.

### Example 7: Binding efficiency of a polymer of the invention on mouse inflamed colon ex vivo

In order to test the ability of the polymer of the invention to bind to inflamed colon the following assay was carried out.

Colitis in mice was induced by feeding the animals with 3% DSS in drinking water over a period of 7 days. The mouse developed an acute colitis, which peaks between day 6-8, and relapses following 14 days. The induction of colitis is monitored by weighing the animals, stool consistency and physical appearance of the mouse. At the final day, animals are anesthetized and a colonoscopy is done. Then, colons are extracted and flushed with a 5 mL syringe attached to 100 µL pipette tip with PBS and opened longitudinally. Colons are then cut into sections and submerged in Carnoy fixation solution for 60 min. Sections are then embedded in Tissue-Tek® O.C.T.™ Compound and frozen. Colon slides (30 µm thickness) are then prepared with the aid of a cryostat. Slides are incubated for 10 min with 5 µM of the polymer of the invention obtained in Example 6 in PBS. Then, slides are washed with PBS three times for 5 min, 5 µL of 4',6-diamidino-2-phenylindole (DAPI) are added to stain the nuclei and further covered with coverslides. Slides are then observed in the microscope. The selective binding to inflamed regions of the polymer of the invention was observed in inflamed tissue as shown on Figure 5 (white circle).

### Example 8: Binding efficiency of a polymer of the invention on swine colon ex vivo

In order to test the specificity of the polymer of the invention to bind to a target in the colon the following assay is carried out.

Polymers of the invention with and without targeting molecules are incubated *ex vivo* along with colon samples obtained from a healthy swine: one polymer of Formula (II) wherein R¹ is TAMRA dye, R² is (CH₂)₃, R³ is (CH₂)₂ and R⁴ is (CH₂CH₂O)₂, R⁵ is Br and R⁶ a targeting molecule for the extracellular domain of the cystic fibrosis transmembrane conductance regulator protein (CFTR) (anti-CFTR antibody, Thermo Scientific, MA1-935) and another polymer of Formula (I), being R¹ TAMRA dye, R² is (CH₂)₃, R³ is (CH₂)₂ and R⁴ is (CH₂CH₂O)₂ and R⁵ is Br, as negative control. In order to perform a click reaction with the polymer of Formula (I), to form a polymer of Formula (II) as referenced above, alkyne moieties are linked to the commercial antibody for further reacting with the azide groups of the polymer. For this purpose, the anti-CFTR antibody is activated by the addition of a linker that bears an alkyne group at the end of the chain (BaseClick™, NHS-PEG-Alkyne, BCL-092-10).

To protect said linker from moisture, the vial of linker (NHS-PEG-Alkyne) is allowed to equilibrate to room temperature before opening. 1 mg of NHS-PEG-Alkyne is dissolved in 100 µL PBS (final concentration: 10 mg/mL, 25 µmol/mL) and 3.5 µL of NHS-PEG-Alkyne solution are mixed with the antibody solution in a 2 mL eppendorf (100 µl of anti-CFTR antibody + 400 µl of PBS) and incubated for 2 hours at room temperature under agitation. Dilute to 1 mL with PBS. The non-reacted NHS-PEG-Alkyne is removed and the activated antibody is concentrated with Vivaspin-2 300kDa MWCO filters (Sartorius). The Vivaspin-2 filter device is centrifuged at 9,000 × g for 15 minutes and the concentrate is washed with PBS solution and centrifuge again at 9,000 × g for 15 minutes. The concentrated solute is then recovered, by adding 100 µl of PBS. After these steps, the antibody is ready to be linked to the polymer as a targeting molecule as described Example 5.

Briefly, after the excision of the colon, the mucosal apical layer is washed with phosphate buffered saline (PBS) to remove the excess of blood and incubated with 1% BSA-PBS solution for 30 minutes, in order to block the further nonspecific binding. After that, the mucosal layer is divided into six sections: three sections are incubated with a 100 µM dye containing polymer of the invention bearing the CFTR targeting molecule and the other three sections are incubated with a 100 µM dye containing polymer of the invention of Formula (I) as defined above (as negative controls) for 10 minutes in the dark at 37°C, in 24-well plates covered with aluminium foil, in order to avoid the light and to mimic the gastrointestinal environment, as described in United States Pharmacopeia 33-28NF (2010) and European Pharmacopeia 7.0 (2010). After this time, the mucosal layer sections are washed three times with PBS for 10 minutes to remove the unbound polymer. In order to detect the polymer of the invention bound specifically to the mucosal layer, a Zeiss StereoLumar.V12 (Carl Zeiss, Oberkochen, Germany) stereomicroscope was used for ex vivo imaging of colon sections. To allow imaging, the following components were installed: a cooled EMCCD camera (Evolve eXcelon, Photometrics, Tuscon, AZ), a high-powered light emitting diode (LED) system with illumination at 470 and 635 (CoolLED, Andover, UK) and specific filters for FITC and Cy5 (Zeiss). Figure 7 shows the representative images of the colon sections in which highly fluorescent areas can be observed when incubated with the CFTR targeting molecule bearing polymer of the invention, whereas no fluorescence signal is observed in the samples incubated with the polymer of the invention without any targeting molecule. Those data support the fact that the binding of a polymer of the invention is very specific and does not present unspecific binding to colon tissue.

### Example 9: Binding efficiency of a polymer of the invention on human inflamed colon ex vivo

In order to test the ability of the polymer of the invention to bind to inflamed human colon the following assay is carried out.

Two different polymers of the invention are incubated *ex vivo* along with colon samples obtained from a colorectal cancer patient: one polymer of Formula (II), being R¹ DyLight™ 633 dye, R² is (CH₂)₃, R³ is (CH₂)₂ and R⁴ is (CH₂CH₂O)₂, R⁵ is Br and R⁶ is a targeting molecule for inflamed tissue and another polymer of Formula (II), being R¹ DyLight™ 633 dye, R² is (CH₂)_{3,} R³ is (CH₂)₂ and R⁴ is (CH₂CH_{2O})₂, R⁵ is Br and R⁶ is a human anti-MUC2 antibody (SantaCruz, sc-13312) in another polymer). As described in Example 8, the anti-MUC2 antibody is activated by the addition of a linker that bears an alkyne group at the end of the chain (BaseClick™, NHS-PEG-Alkyne, BCL-092-10) under the same procedure.

Briefly, after the excision of the inflamed human colon, the mucosal apical layer is separated from the lamina propria and the muscularis mucosae with the help of surgical scissors and forceps. The mucosal layer is then washed with phosphate buffered saline (PBS) to remove the excess of blood and incubated with 1% BSA-PBS solution for 30 minutes, in order to block the further nonspecific binding. After that, the mucosal layer is incubated with a 100 µM dye containing polymer of the invention (being R⁶ a targeting molecule for inflamed tissue) for 10 minutes in the dark at 37°C, in a 50 mL tube covered with aluminium foil, in order to avoid the light and to mimic the gastrointestinal environment, as described in United States Pharmacopeia 33-28NF (2010) and European Pharmacopeia 7.0 (2010). After this time, the mucosal layer is washed three times with PBS for 10 minutes to remove the unbound polymer. In order to detect the polymer of the invention bound specifically to the mucosal layer, a Zeiss StereoLumar.V12 (Carl Zeiss, Oberkochen, Germany) stereomicroscope was used for ex vivo imaging of colon sections. To allow imaging, the following components were installed: a cooled EMCCD camera (Evolve eXcelon, Photometrics, Tuscon, AZ), a high-powered light emitting diode (LED) system with illumination at 470 and 635 (CoolLED, Andover, UK) and specific filters for FITC and Cy5 (Zeiss). Figure 6 shows the image of the colon section in which highly fluorescent areas can be observed. Those data, along with the lack of unspecific binding shown in Example 8, support the fact that the binding of a polymer of the invention is very specific.

### Example 10: Controlling lack of absorption of a polymer of the invention in mice in vivo

In order to confirm lack of absorption of polymers of the invention, a polymer of the invention with a targeting molecule marked with radioactive isotope ^{99m}Tc (polymer of Formula (I') of Example 6) as described in Inchaurraga et al., 2014, Eur J Pharm Biopharm, in press (doi:10.1016/j.ejpb.2014.12.021*),* and is orally administered to both colitis-suffering (inflamed) and colitis-non-suffering (healthy, control) mice. After 4 h and 24 h, radioactivity signal were measured from the mice to assess the absorption of the polymer of the invention. After 24 h mice were euthanized and tissues were harvested and analyzed in order to study the possible biodistribution of the polymer. Figure 8 shows how in both healthy and inflamed mice groups there is no spread radioactive activity, meaning that all radioactive signal is concentrated in the lower GI tract, without being absorbed at 4h. This is also confirmed by the radioactive values data obtained from the harvested organs at 24 h and summarized in Table 8, where the mean ± SD of %ID (% of the injected dose) in organs are expressed. All values were less than 0.1 %ID.

**Table 8**

| | Non-colitis | Colitis |
|---|---|---|
| Blood 4h | 0.013 ± 0.007 | 0.063 ± 0.008 |
| Blood 24h | 0.002 ± 0.001 | 0.017 ± 0.005 |
| Liver 24h | 0.005 ± 0.002 | 0.010 ± 0.002 |
| Heart 24h | 0 | 2.34 x 10-4 |
| Kidney 24h | 0.003 ± 0.001 | 0.006 ± 0.001 |

Altogether those data support the fact that a polymer of the invention is not absorbed in the gastrointestinal tract. Furthermore, in Figure 8 it can also be seen that while in the healthy group there is no radioactive signal after 24 h in the lower GI tract, a residual radioactive activity can be observed in the intestine of the inflamed mice group, suggesting the fact that a polymer of the invention can be used as a suitable tool to target a specific tissue, in particular inflamed colon tissues, as it already had been observed in Examples 8 and 9.

## Claims

1. A block copolymer of methacrylate based monomers comprising at least one azide bearing methacrylate monomer and at least one another methacrylate monomer selected from sulfonated methacrylate monomer and hydroxylated methacrylate monomer.

2. A copolymer according to claim 1 wherein the copolymer is a copolymer of ω-azido oligoethyleneglycol methacrylate (AOEGMA) with at least another monomer selected from 2-sulfoethyl methacrylate (SEMA) and 3-sulfopropyl methacrylate (SPMA).

3. A copolymer according to claim 1 or 2 further comprising a monomer selected from hydroxyethyl methacrylate (HEMA) and hydroxypropyl methacrylate (HPMA).

4. A di- or tri-block copolymer according to any one of claims 1 to 3 of ω-azido oligoethyleneglycol methacrylate (AOEGMA) with 3-sulfopropyl methacrylate (SPMA) and optionally hydroxyethyl methacrylate (HEMA).

5. A block copolymer according to any one of claims 1 to 4 having the following Formula (I): 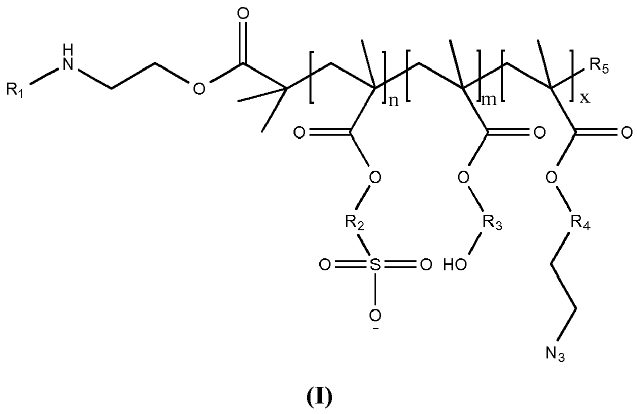 wherein n is an integer from about 20 and 550, m is an integer from about 0 and 1037 and x is an integer from 10 and 310, R¹ is selected from a blocking group, N-hydroxysuccinimide (NHS) ester, a maleimide group, a hydrazide group, a hydrogen atom, an amine group and a dye; R², R³ and R⁴ are independently selected from (CH₂)ₐ, benzyl and (CH₂CH₂O)_{b}, wherein a is an integer from 1 and 6, b is an integer from 1 and 8; and R⁵ is selected from a halogen and H.

6. A block copolymer according to any one of claims 1 to 4 wherein R¹ is a biocompatible dye.

7. A block copolymer according to any one of the preceding claims wherein the polymer has a molecular weight of about 50 kDa to about 500 kDa.

8. A tri-block copolymer according to any one of the preceding claims wherein n is 246, m is157 and x is 33, wherein the polymer has a molecular weight of about 89 kDa.

9. A tri-block copolymer according to any one of the preceding claims having the following formula (I'):

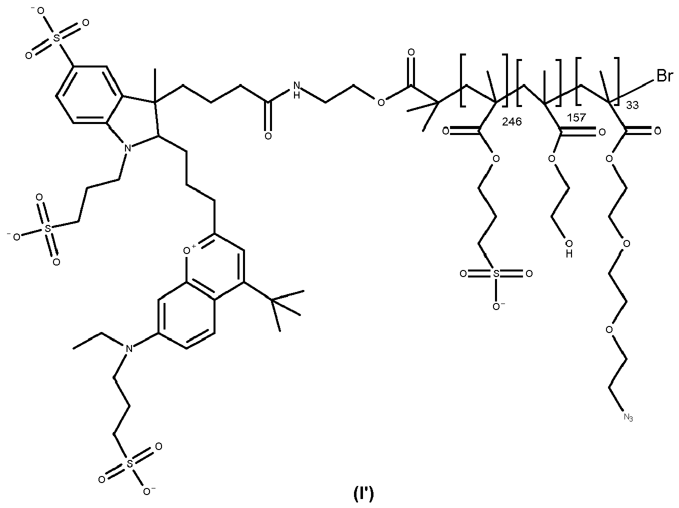

10. A method of preparation of a diagnosis probe comprising a step of coupling a block copolymer of according to any one of claims 1 to 9 with at least one targeting molecule.

11. A method according to claim 10 comprising the following steps:
(i) Providing a block copolymer of according to any one of claims 1 to 9;
(ii) Reacting the said polymer with an alkyne bearing targeting molecule under an azide-alkyne Huisgen cycloaddition;
(iii) Isolating the resulting block copolymer.

12. A method of preparation of a diagnosis probe according any one of claims 10 to 11 wherein the resulting polymer is a block copolymer according to Formula (II): 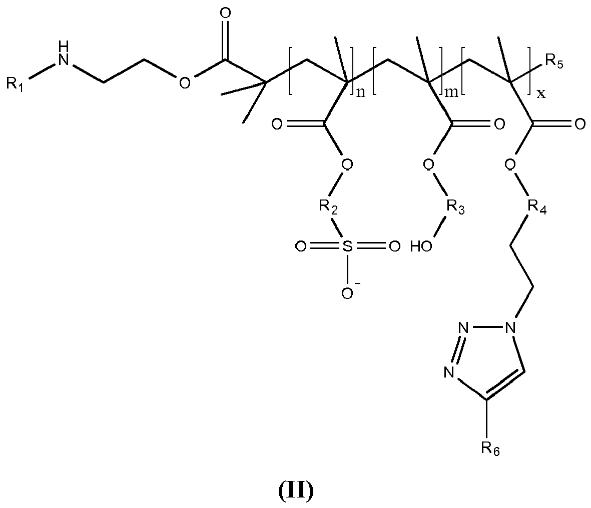 wherein m, n, x, R¹. R² R³, R⁴, R⁵ are as defined in anyone of the preceding claims and R⁶ is a targeting molecule.

13. A diagnosis probe obtainable from a method according to claims 10 to 12.

14. A diagnosis probe according to Formula (II) wherein R¹ is selected from a blocking group, N-hydroxysuccinimide (NHS) ester, a maleimide group, a hydrazide group, a hydrogen atom, an amine group and a dye; R², R³ and R⁴ are independently selected from (CH₂)ₐ, benzyl and (CH₂CH₂O)_{b}, wherein a is an integer from 1 and 6, b is an integer from 1 and 8; m, n, x and R⁶ are as defined in anyone of the preceding claims.

15. A targeting molecule of Formula (III) or a fragment or variant thereof:

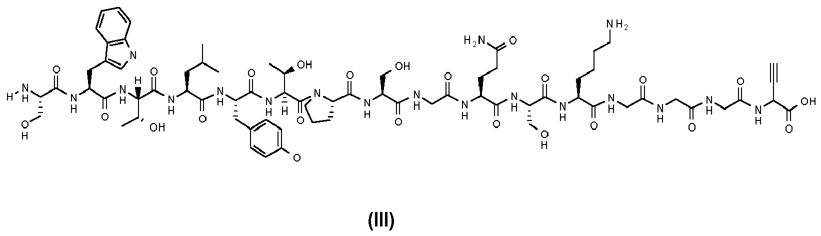

16. A block copolymer according to claims 1 to 9 or a diagnosis probe according to claims 13 to 14 or a targeting molecule according to claim 15 for use as a medicament or as a diagnosis agent.

17. A pharmaceutical composition comprising at least one block copolymer according to anyone of claims 1 to 9 or a diagnosis probe according to claims 13 or 14 and at least one pharmaceutically acceptable carrier, diluent or excipient thereof.

18. A block copolymer according to claims 1 to 9 or a diagnosis probe according to claims 13 to 14 or a targeting molecule according to claim 15 or a pharmaceutical composition thereof for use in the early detection, diagnosis or prevention and/or treatment of a disease or disorder such as inflammatory bowel diseases (IBDs) or a cancer such as colorectal cancer.
